# NEUE EUROPÄISCHE PATENTSCHRIFT

(11) **EP 2 223 995 B2**
(45) Veröffentlichungstag und Bekanntmachung des Hinweises auf die Entscheidung über den Einspruch: **11.12.2024**
(45) Hinweis auf die Patenterteilung: 07.03.2018
(21) Anmeldenummer: 10004122.7
(22) Anmeldetag: 19.09.2008
(51) Int. Cl.: C11D 17/00, C11D 3/20, C11D 3/18, C11D 3/50, C11D 11/00, C09D 9/00, C09D 9/04, A61Q 3/04, A61Q 5/02

(54) **FORMULIERUNGEN ZUR ENTFERNUNG VON FARBSCHICHTEN UND DIVERSEN SCHMUTZSCHICHTEN VON OBERFLÄCHEN, VERFAHREN ZUR HERSTELLUNG DES MITTELS UND VERFAHREN ZUR REINIGUNG**
CLEANING AGENT FOR REMOVING PAINT LAYERS ON SURFACES, METHOD FOR MANUFACTURING THE AGENT AND CLEANING METHOD
PRODUIT DE NETTOYAGE POUR LA SUPPRESSION DE COUCHES DE COULEURS DE SURFACES, PROCÉDÉ DE FABRICATION DU PRODUIT ET PROCÉDÉ DE NETTOYAGE

(30) Priorität: 19.09.2007 EP 07116792
(43) Veröffentlichungstag der Anmeldung: 01.09.2010
(62) Teilanmeldung aus: 08804501.8
(73) Patentinhaber: intelligent fluids GmbH, 04229 Leipzig (DE)
(72) Erfinder: Schumann, Dirk, 04229 Leipzig (DE); Surkow, Rainer, 04105 Leipzig (DE)
(74) Vertreter: Wetzel, Fritz

(56) Entgegenhaltungen:
- EP-A1- 0 670 380
- EP-A1- 1 063 283
- EP-A1- 1 388 575
- EP-A1- 1 770 446
- EP-A2- 0 316 726
- EP-A2- 1 582 196
- WO-A1-2004/078897
- WO-A1-2007/133934
- WO-A1-91/00337
- WO-A1-92/18600
- WO-A1-96/16132
- WO-A1-99/06520
- WO-A1-99/58631
- WO-A2-2009/037349
- CH-A5- 670 832
- DE-A1- 10 049 831
- DE-A1- 102004 010 152
- DE-A1- 102005 062 175
- DE-A1- 4 406 753
- DE-B- 1 236 704
- DE-T2- 69 029 472
- FR-A1- 2 438 078
- GB-A- 1 317 773
- GB-A- 2 190 681
- US-A- 4 606 840
- US-A- 4 659 332
- US-A- 5 158 710
- US-A- 5 281 354
- US-A- 5 346 640
- US-A- 5 451 405
- US-A- 5 536 450
- US-A- 5 604 195
- US-A- 5 712 343
- US-A- 5 753 603
- US-A- 5 952 287
- US-A- 5 962 383
- US-A- 6 057 279
- US-A- 6 071 867
- US-A- 6 093 410
- US-A1- 2002 010 104
- US-A1- 2003 082 912
- US-A1- 2003 219 465
- US-A1- 2004 259 753
- US-A1- 2005 130 869
- US-A1- 2005 232 953
- US-A1- 2006 084 589
- US-A1- 2006 165 739
- US-A1- 2007 269 467
- US-B1- 6 555 119
- US-B1- 6 569 414
- US-B1- 6 689 727
- LEKKERKERKER ET AL.: "Phase Behavior of Ionic Microemulsions", BER. BUNSENGES. PHYS. CHEM., vol. 100, no. 3, 1996, pages 206 - 217

## Beschreibung

Die vorliegende Erfindung beschreibt neue Reinigungsmittel. Das erfindungsgemäße Reinigungsmittel dient insbesondere zur Entfernung von Graffiti von glatten und/oder porösen Untergründen, zum Abbeizen von Lacken von Metall-, Holz- oder Glasoberflächen, zur Schmutzentfernung, zur Entfernung von Kosmetik, zur Haarentfärbung und zur Entfernung von Nagellack. Die Reinigungswirkung der erfindungsgemäßen Mittel besteht dabei in einer Fragmentierung der Schmutz- oder Farbschichten und deren Ablösen vom Untergrund, wobei ein Verschmieren weitgehend vermieden wird. Ermöglicht wird dies durch die erfindungsgemäße Zusammensetzung speziell erweiterter Mikroemulsionen.

Anfang der siebziger Jahre breitete sich, von New York ausgehend, im Zuge der Hip-Hop Kultur das Besprühen und Bemalen von Wänden und anderen Oberflächen aus. Nach einer Schätzung des Deutschen Städtetages (April 2002) beläuft sich der volkswirtschaftliche Schaden durch Graffiti in Deutschland auf etwa 200 Millionen Euro, hinzu kommen etwa 50 Millionen bei den U- und S-Bahnen und der Deutschen Bahn. Auch wenn in vielen Fällen gut ausgearbeitete Graffiti (sog. "High-Graffiti" oder "Masterpiece") eine Kunstform darstellen, handelt es sich im deutlich überwiegenden Teil um Schmierereien • (sog·. "Tags") und damit um Sachbeschädigung. Die bisherigen kommerziell erhältlichen Graffitientferner helfen nur bedingt oder gar nicht. Sie sind meist nur auf glatten Flächen wirksam und benötigen eine lange Einwirkzeit von in der Regel 30 Minuten bis zu 2 Stunden. In gewissen Fällen wird sogar eine Einwirkzeit von bis zu 24 Stunden und eine anschließende Behandlung mit einem Hochdruckreiniger mit 90 °C heißem Wasser mit einem Druck von 150 bar empfohlen.

Die Anforderungen an einen Graffitientferner sind sehr hoch, da das Mittel auf unterschiedlichen Untergründen wirken muss und Graffitifarben zudem sehr uneinheitlich in ihrer Zusammensetzung sind. Graffiti-Farben und Beschichtungen besitzen keine einheitliche Zusammensetzung, d.h. die Bindemittel variieren je nach Herstellerfirma, dem Anwendungszweck der Farbe und selbst innerhalb von Artikelserien. Nähere Auskünfte der Herstellerfirmen zu den Bestandteilen ihrer Farbprodukte werden in der Regel nicht erteilt.

Die besprühten oder bemalten Untergründe sind meist aus Beton, Putz, Ziegel und Klinker, aber auch aus Feinkeramik und Natursteinen, wie Marmor oder Sandstein. Bei porösen Untergründen dringen die Farben in die Poren und Mauerfugen ein, so dass ein Entfernen nur schwer möglich ist. Einfacher zu reinigen sind glatte Oberflächen, wie lackierte oder unlackierte Metalle, Kunststoffe oder Glas.

Neben dem Untergrund entscheiden auch die Farbpigmente über den Aufwand der Farbentfernung. Zum einen hinterlassen dunkle Farbtöne bereits rein optisch einen auffälligen Farbschatten auf hellen Wänden, zum anderen werden feinere Pigmentpartikel leichter in Poren eingetragen und verbleiben dann darin. Farbschatten werden teilweise auch durch Pigmente mit ungenügender "Echtheit" erzeugt. Hier sind die Pigmente nicht völlig unlöslich, so dass Pigment-Moleküle z.B. in einen lackierten Untergrund oder durch einen Neuanstrich migrieren und führen u.a. zum sog. Durchbluten".

Zu den feinteiligen Pigmenten gehören v.a. die Ruße (schwarz) und Phthalocyanine (grün bis blau) sowie einige andere organische Pigmente. Bei einfach aufgebauten organischen Pigmenten finden sich auch häufig solche, die zum Durchbluten neigen. In der Regel besitzen die weißen, gelben und roten Pigmente auf mineralischer Basis (Titandioxid, Eisenoxide etc.), Ultramarin und Metallic-Pigmente (Aluminium, Bronzen) größere Partikeldurchmesser und lassen sich daher leichter aus dem Bindemittel herauslösen.

Die eingesetzten Bindemittel der Graffitifarben können Alkydharze sein mit unterschiedlichem Aufbau (z.B. Polyester- bzw. Fettsäureart und -gehalt), Harze, modifizierte oder vollsynthetische Kautschuke, Celluloseester, Aldehydkondensate oder viele anderen Polymerisate. Bei lösungsmittelfreien, wassermischbaren Sprayfarben sind es häufig· Acryl- oder Polyvinylacetat-Dispersionen, manchmal auch alkalisch gelöste Acrylharze.

Die Graffitientfernung ist ein spezieller Fall der Lackentfernung und des Abbeizens. Die Aufgabe der Lackentfernung bei gleichzeitigem Schutz des Untergrundes ist in beiden Fällen gleich. Im Gegensatz zur Graffitientfernung ist die Lackentfernung oder das Abbeizen allerdings mit weniger schwierigen, d.h. porösen oder stark strukturierten Untergründen verbunden, dafür müssen hier oft auch vernetzte Lacksysteme oder koaleszierte Hochpolymerpartiken aus Dispersionsfarben entfernt werden, die sich durch eine Unlöslichkeit in Lösungsmitteln und in einer besonders guten Haftung zum Untergrund auszeichnen.

Seit Jahrzehnten wurden für die Lackentfernung universell wirkende Formulierungen verwendet, nämlich starke Lösungsmittel, allen voran Dichlormethan. Auf Grund der Toxizität dieses chlorierten Kohlenwasserstoffs und einem verstärkten Umweltbewusstsein der Gesellschaft sind diese Lösungsmittel und verwandte Kohlenwasserstoffe heutzutage nicht mehr ohne weiteres einsetzbar. So beschäftigen sich in Deutschland die Technischen Regeln für Gefahrstoffe TRGS 6123 eingehend mit möglichen Ersatzstoffen und Verfahren für Dichlormethan. Alternative halogenfreie Abbeiz-Formulierungen haben häufig den Nachteil, dass sie länger Einwirkungszeiten benötigen, teilweise hochgradig entflammbar sind und nicht bei jedem Lacksystem ein gleichermaßen gutes Ablösen zeigen. Gerade die letztgenannte Problemstellung zeigt sich heutzutage häufiger, da immer mehr wasserbasierte Lackformulierungen auf den Markt kommen, die gänzlich andere Solubilisierungseigenschaften erwarten, als die bisher verwendeten Lösungsmittel- basierten Lacksysteme. Für die heutige Vielzahl an Lacksystemen werden also Formulierungen zur Lackentfernung benötigt, die sowohl hydrophile wie auch hydrophobe Lösungseigenschaften aufweisen. Die Lackentfernung betrifft im übrigen nicht nur das Ablösen von aufgetrockneten Lackschichten, sondern auch die Entfernung von frischen Lackformulierungen, wie sie in weiten Anwendungsfällen auftreten, vom Malerhandwerk bis zur Druckfarbenindustrie.

Ein spezielles Gebiet der Lackentfernung ist die Entfernung von Nagellack. Bei der Entfernung von kosmetischen Nagellacken tritt infolge der Einwirkung von gebräuchlichen organischen Lösungsmitteln in den weitaus meisten Fällen eine Entfettung und ein Auslaugen der Nägel sowie der umliegenden Hautpartien auf. Dies macht sich in Form eines Weißwerdens von Nägeln und Haut bis hin zu brüchigen und glanzlosen Nägeln bei der Daueranwendung von Nagellackentferner bemerkbar. Seit vielen Jahrzehnten werden daher bereits verschiedene Vorschläge gemacht, um diese Effekte zu vermeiden oder gar zu verhindern. Obwohl es bekannt ist, dass die Entfettung und der Entzug von wichtigen Aufbaustoffen aus dem Nagel durch die Lösungsmittel erfolgt, wurden und werden immer noch Aceton, Ethylacetat, Butylacetat und ähnliche in Nagellackentfernern eingesetzt.

Um Graffiti zu entfernen, werden im Stand der Technik neben mechanischen Verfahren wie Sandstrahlen, Trockeneisstrahlen oder der Laserablation meist flüssige oder gelartige Graffitientferner verwendet. Der Vorteil der Graffitientferner ist die schnelle Möglichkeit eines Auftrags und die dann beginnende gleichzeitige Wirkung auf das Ablösen der Farbe auf größeren Flächen, während mit den mechanischen nur lokal die Farbe abgelöst werden kann. Die bisherigen Graffitientferner enthalten einzelne Lösungsmittel oder Lösungsmittelgemische, die die Aufgabe haben, die Bindemittel des Graffiti-Lacks physikalisch zu lösen. In einigen Graffitientfernern sind die Lösungsmittel in Wasser emulgiert. Die oft dünnflüssigen Lösungsmittel oder Emulsionen werden dabei gegen ein Ablaufen an senkrechten Oberflächen meist durch Additive verdickt.

Typisch für konventionelle Graffitientferner sind nachfolgende Lösungsmittel, die alle in der Liste der Technischen Regeln für Gefahrstoffe (TRGS 612, März 2002) zu finden sind. Häufig sind es Ester, wie z.B. kürzere und/oder längere Fettsäureester, wie z.B. Methyloleat bzw. Fettsäuregemische wie Rapsölfettsäuremethylester ("Biodiesel"), sowie verschiedene Dicarbonsäureester, z.B. sog. Dibasenester (DBE- Ester). EP 1772 496 A1 verwendet solche Methylester, wie auch Cyclohexanon, teilweise in (Mikro-)Emulsion zum Auflösen der Graffitifarben. Ab und zu werden auch Milchsäureester eingesetzt. Verbreitet ist auch die Verwendung von Glykolderivaten, wie Propylencarbonat, 1-Methoxy-2-propanol oder 2-Methoxy-1-methylethylacetat.

Strukturell ähnlich ist hier auch das in der Offenlegungsschrift DE 10 2004 015 092 A1 beschriebene Ethyl-3-ethoxypropionat. Ethyl-3-ethoxypropionat wurde aber auch bereits schon viele Jahre zuvor in DE 691 28 887 T2 (EP O 551 378 B1) zur Aktivierung der stark polaren, aprotischen Lösungsmittel 1-Methyl-2-pyrrolidon (NMP) und Gamma-butyrolacton eingesetzt. Gerade das 1-Methyl-2-pyrrolidon ist ein bekanntes und ausgezeichnetes Lacklösemittel, welches entweder in reiner Form oder in Mischungen mit anderen Lösemitteln in vielen Abbeizer sowie Graffitientfernern eingesetzt wird. So beschreibt DE 695 21 333 T1 den Einsatz von 1-Methyl-2-pyrrolidon, aber auch anderen Pyrrolidonen in Mengen von 1 bis 60 Gew.-% in der Formulierung. Auch das dem Gamma-butyrolacton strukturell nahestehende Tetrahydrofuran wird in Graffitientfernern verwendet. Um 1-Methyl-2-pyrrolidon zu ersetzen wird in der Offenlegungsschrift DE 10 2004 012 751 A1 das 1-Ethyl-2-pyrrolidon beschrieben, welches auch bereits schon Bestandteil von DE 695 21 333 T1 war. Als strukturell sehr ähnlich zu den Pyrrolidonen kann auch das N-Methylcaprolactam aus Offenlegungsschrift DE 102004015182 A1 angesehen werden.

Dichlormethan und Gamma-butyrolacton sind aufgrund ihrer umwelt- bzw. gesundheitlich bedenklichen Wirkung zu vermeiden. Aber auch 1-Methyl-2-pyrrolidon ist in der jüngsten Zeit in Verdacht geraten teratogen zu wirken. Viele Graffitientferner und Abbeizer beinhalten oft größere Mengen an NMP, da es für die Entlackung hervorragende geeignet ist und bis vor kurzem als toxikologisch wenig bedenklich galt. Unabhängig davon, ob sich die Verdachtsmomente in Zukunft erhärten, ist sein vollständiger Ersatz auch aus Sicht der Produktvermarktung sinnvoll.

Kurzkettige Lösungsmittel-Moleküle dringen oft schnell in die Bindemittel der Graffiti- Farben ein, verdunsten in den meisten Fällen aber auch wieder schnell. Langkettige Lösungsmittel-Moleküle benötigen für diesen Vorgang häufig wesentlich mehr Zeit (z.B. zwischen etwa 20 Minuten bis 2 Stunden) und führen zu einem Anquellen der Beschichtungen, so dass diese danach mechanisch leichter vom Untergrund abgerieben werden können.

Nachteilig an einzelnen Lösungsmitteln und selbst bei Lösungsmittelgemischen ist, dass aufgrund der Komplexität des Lösevorgangs, welcher bis heute besonders im Hinblick auf das Lösen von Lacken oder Polymeren noch nicht komplett in seinen Einzelheiten verstanden ist, nicht alle Bindemittel-Typen gleichermaßen gut gelöst werden können. Dies macht sich dann bemerkbar, wenn man versucht verschiedene Graffiti-Farben mit demselben Entfemer-System zu lösen. Aus einer breiten Palette von verschiedenen Aerosol-Farben lassen sich immer einige davon nicht zufriedenstellend vom Untergrund ablösen.

Selbst im Fall eines gelungenen Lösevorgangs besteht ein weiterer Nachteil von Lösungsmitteln darin, dass das Bindemittel der Graffiti-Farbe stark verdünnt und mitsamt den Farbpigmenten über den ursprünglichen Rand des Graffito hinaus verteilt wird. Sofern der Untergrund nicht glatt ist, sondern eine strukturierte Oberfläche besitzt, bleiben in vielen Fällen Reste des Bindemittels und v.a. die Farbpigmente in feinsten Ritzen und Poren hängen. Dies macht sich später in Form eines farbigen "Schattens" bemerkbar, der nur sehr schwer bis überhaupt nicht mehr zu entfernen ist. Der Untergrund muss wohlgemerkt dabei gar nicht porös und saugfähig wie die meisten Putze an Hauswänden sein, auch an glasierten rauen Kacheln, Keramiken oder Klinkern lässt sich diese Schattenbildung beobachten.

Da man bei zu entfernenden Graffiti-Farben am Schadensort nicht die Pigment- Bindemittel-Zusammensetzung erkennen kann, muss ein Graffitientferner ein möglichst breites Spektrum an Lösungsfähigkeit besitzen. Wie oben angemerkt können dies auch Lösungsmittel-Gemische nicht vollständig erzielen. Es muss also ein System gefunden werden, womit sich eine große Anzahl an unterschiedlichen Bindemitteln vom Untergrund ablösen lassen. Das System sollte die Graffitifarben in einer kurzen Zeit, z. B. innerhalb von 10 Minuten, maximal bis zu einer halben Stunde anlösen und vom Untergrund möglichst ohne Farbschatten entfernen können. Der Graffitientferner sollte keine giftigen oder langfristig gesundheits- bzw. umweltproblematischen Stoffe enthalten, namentlich keine organischen Halogen-Verbinpungen (wie Dichlormethan), kein Gamma-Butyrolacton und möglichst auch keine N-Alkyl-pyrrolidone, aber auch keine starken Säuren oder Basen.

Ebenso wie bei Graffitientfernern sollten Abbeizer keine giftigen oder langfristig gesundheits- bzw. umweltproblematischen Stoffe enthalten, namentlich keine organischen Halogen-Verbindungen wie Dichlormethan, kein Gamma-Butyrolacton und möglichst auch keine N-Alkyl-pyrrolidone. Ebenso sollten starken Säuren oder Basen vermieden werden. Die breite Anwendbarkeit und die schnelle Wirkung gelten bei Dichlormethan-haltigen Abbeizern immer noch als vorteilhaft. Dichlormethan- freie Mittel benötigen jedoch oft deutlich längere Einwirkungszeiten, teilweise bis über 24 Stunden. Auch wenn dadurch andere größere Tiefenwirkung erzielt wird, sind solche langen Zeiträume inakzeptabel. Daneben bleibt das Problem des Verschmierens durch den Einsatz von Lösungsmitteln oder deren Gemischen. Ein geeigneter Abbeizer sollte daher nicht umwelt- und gesundheitsgefährdend sein, das heißt keines der oberen genannten Lösungsmittel, Säuren oder Basen enthalten, in möglichst kurzer Zeit auf eine sehr breite Palette an Farben und Bindemittel wirken, kein Verschmieren der Altlacke herbeiführen, die verschiedene Untergründen nicht angreifen und möglichst mit Wasser entfernbar sein.

Wie im Fall der Graffiti-Entfernung und Lackentfernung ist auch bei der Nagellack-Entfernung - hier jedoch durch die kosmetische Anwendung im Besonderen - die Auswahl an möglichst wirkungsvollen einerseits und möglichst umwelt- und gesundheitsschonenden Inhaltsstoffen andererseits notwendig. Der Einsatz von Lösungsmitteln wie Dichlormethan (DE 1089515) oder Tetrachlormethan (DE 830094) bzw. ähnlich halogenierte Kohlenwasserstoffe ist in heutigen Formulierungen undenkbar. Aber auch über lange Zeit in Nagellackentfernern verwendete Lösungsmittel, wie z.B. gamma-Butyrolacton (GBL) existieren seit kurzer neue Erkenntnisse über ihre negativen Auswirkungen auf die Gesundheit.

Der moderne Verbraucher erwartet von einem Nagellackentferner mittlerweile nicht nur die Funktionen der Reinigung und Pflege von Nägel und Hautpartien, sondern auch unbedenkliche und sensorisch dezente Inhaltsstoffe. Gerade in den letzten Jahren ist eine zunehmende Sensibilisierung der Verbraucher gegenüber den stechenden oder scharfen Gerüchen von Lösungsmitteln in einer Vielzahl von Produkten aufgetreten. Dies trifft insbesondere für die am häufigsten in Nagellackentfernern verwendeten Lösungsmittel zu, wie Aceton, Ethylacetat, Butylacetat, Ethyllactat, etc.

Auch wenn sich diese Lösungsmittel in Nagellackentfernern durch ihre schnelle Wirksamkeit und relativ geringe Toxizität bewährt haben, so ist doch ihr Ersatz auf Grund der charakteristischen Gerüche und der immer noch bestehenden Problematik der Haut- und Nagel-Entfettung dringend angeraten. Der mittlerweile in so genannten umweltverträglichen Produkten enthaltene Bio-Ethanol ist wiederum keine geeignete Alternative als Lösungsmittel, da sich nur spezielle Nagellacke damit entfernen lassen, so dass ein komplett neues Wirksystem gefunden werden muss, das alle oben genannte Ansprüche erfüllt.

Ein Nagellackentferner sollte also in der Lage sein, die aufgetragenen Beschichtungen schnell und sauber vom Nagel zu entfernen, ohne die oben genannten Schadensbilder zu verursachen. Stark entfettende Lösungsmittel sollten nicht oder nur in geringen Mengen eingesetzt werden, rückfettende und Feuchtigkeit spendende Substanzen sollten in ausreichender Menge zur Verfügung stehen, dürfen aber nicht die Entfernung des Lackes behindern oder verzögern. Wie im Fall von Aerosol-Farben und anderen Farbmitteln, mit denen Graffiti erzeugt werden, existieren eine Reihe von polymeren Lackbindemitteln die in den Formulierungen von Nagellack Anwendung finden. Ein geeigneter Nagellackentferner muss neben der Nagelpflege auch in der Lage sein, alle diese unterschiedlichen Lackzusammensetzungen gleichermaßen gut vom Untergrund abzulösen. Ebenso wie im Fall des Graffitientferners, jedoch in diesem Anwendungsfall von etwas geringerer Bedeutung, sollten beim Einsatz des Nagellackentferners die Farbmittel nicht verschmiert werden.

Aufgabe der vorliegenden Erfindung war es also, ein Reinigungsmittel für eine breite Palette baulicher Untergründe und auch für Haut, Haare und Fingernägel zur Verfügung zu stellen, das ein schnelles und gründliches Entfernen der Farbe oder des Lackes ohne Verschmieren gewährleistet. Das Reinigungsmittel sollte nicht umwelt- oder gesundheitsgefährdend sein und eine breite Palette an Farben und Lacken lösen.

Die Aufgabe der Erfindung wird gelöst durch Bereitstellung eines Reinigungsmittels umfassend ein nicht koaleszierendes, thermodynamisch stabiles Nanophasensystem, wie in Anspruch 1 dargestellt.

In einer bevorzugten Ausführungsform kann das Reinigungsmittel eine weitere amphiphile Substanz umfassen.

Mehrkomponentensysteme vom Typ Wasser, wasserunlösliche Substanz (Öl), Tensid und ggf. Cotensid, die sich spontan bilden und als Mehrstoffsysteme in Erscheinung treten, sind als Mikroemulsionen bekannt. Mikroemulsionen sind thermodynamisch stabile, nanostrukturierte Fluide, die zumindest aus Wasser oder einer wasserähnlichen Flüssigkeit (z.B. Glycerin), Öl und einem Tensid bestehen. Mikroemulsionen enthalten teilweise noch Cotenside und (bei der Verwendung ionischer Tenside) ggf. noch Salze. Die Strukturgrößen der Mikroemulsionen liegen meist zwischen 10 bis 200 nm. Im Gegensatz zu den kinetisch stabilen Emulsionen oder Nanoemulsionen neigen die thermodynamisch stabilen Mikroemulsionen nicht zum Aufrahmen durch Partikelkoaleszenz. In Mikroemulsionen zerfallen kurzfristig entstandene größere Strukturen einige Zeit später wieder in kleinere Mizellen. Daraus folgt, dass sich Mikroemulsionen durch ihre thermodynamische Stabilität auch ohne Durchmischen von selbst bilden. Im Gegensatz zu Emulsionen treten in Mikroemulsionen nicht nur kugelförmige Mizellen, sondern auch elongierte Mizellen (wurmartige Mizellen) und diverse Netzwerk-artige Strukturen auf. Im günstigsten Fall existiert in einer Mikroemulsion eine bikontinuierliche Struktur. Hier durchdringen sich Wasser- und Ölphase über Schwamm-ähnliche Grenzflächen aus Tensiden und ggf. Cotensiden.

Durch die erfindungsgemäße weitere Zugabe von amphiphilen Substanzen, sogenannten NP-MCA (nanophase-forming mixed-chain structure amphiphile}, die nicht der hydrophilhydrophoben Struktur oder Eigenschaften von Tensid bzw. Cotensid folgen, kann eine Erweiterung des einphasigen kolloiddispersen Bereichs der Mikroemulsion erreicht und eine Veränderung der Eigenschaften eingestellt werden.

Überraschend wurde ferner festgestellt, dass die Zugabe von NP-MCAs eine Erweiterung des thermodynamisch stabilen, einphasigen Existenzbereichs der nanostrukturierten Systeme bewirkt. Das war um so überraschender, da die Fachwelt bisher davon ausgegangen war, dass je unterschiedlicher die lipophilen und die hydrophilen Teile hinsichtlich ihrer Löslichkeit in der jeweiligen entgegengesetzten Phase sind, desto eher können sich Mikroemulsionen ausbilden. Daher hat der Fachmann zur Herstellung so genannter Mikroemulsionen grundsätzlich Öle und hydrophile Bestandteile genommen, die sich möglichst wenig ineinander lösen. Folglich wurden gemäß dem Stand der Technik solche Stoffe zur Herstellung von Mikroemulsionen gemieden, die nicht grenzflächenaktiv sind und sich dennoch sowohl in der Ölphase also auch in der hydrophilen Phase aufhalten, wie es den erfindungsgemäßen nichtstrukturbildenden, gemischt-strukturierten Amphiphilen (NP-MCA) der Fall ist.

Insofern überwindet die vorliegende Erfindung ein seit langem in der Fachwelt verwurzeltes Vorurteil.

Es war des Weiteren überraschend, dass die Zugabe von NP-MCAs zu einer Öl/Wasser/Tensid-Mischung eine deutliche Aufweitung des Einphasenbereichs der" entstandenen Nanophasenfluiden gegenüber herkömmlichen Mikroemulsionen entstehen lässt und, gegenüber herkömmlichen Mikroemulsionen, die lamellare Phase (La) in einem als Fisch-Diagramm oder "whale-diagram" bezeichneten Phasendiagramm weit zurückgedrängt wird, so dass das Auftreten von hochviskosen lamellaren Phasen, in denen die Öl- und Wasserdomänen nachteilig schichtweise vorliegen, verhindert oder zumindest vermindert wird (siehe Fig. 10).

Auch war es überraschend, dass durch die erfindungsgemäße Zugabe eines NP-MCA, beispielsweise eines Ethylacetoacetats, eine Absenkung des Temperaturfensters erfolgt und somit ein gegenüber herkömmlichen Mikroemulsionen größerer nutzbarer Temperaturbereich erreicht werden kann (siehe Fig. 10).

Diese Systeme werden im Sinne der Erfindung als fluide Nanophasensysteme (kurz: Nanophasenfluide) bezeichnet. Nanophasenfluide enthalten Wasser oder einen wasserähnlichen Stoff, Öl, mindestens ein strukturbildendes Amphiphil, das sich an die Öl-Wasser-Grenzfläche anlagert und - in Erweiterung zu den Mikroemulsionen - mindestens ein weiteres nicht-strukturbildendes Amphiphil ohne Tensidstruktur (NP-MCA). Das strukturbildende Amphiphil ist ein Tensid, Cotensid oder ein Tensid-ähnliches Oligomer bzw. Polymer. Die NP-MCAs sind wichtig für die Erweiterung des thermodynamisch stabilen Existenzbereichs der fluiden Nanophasen und daher ein weiteres Abgrenzungskriterium zu den Mikroemulsionen.

Die Zugabe von NP-MCAs ermöglicht eine deutliche Aufweitung und ggf. Absenkung des Temperaturfensters des Einphasenbereichs. Daneben verhindern oder vermindern NP-MCAs das Auftreten von hochviskosen lamellaren Phasen, setzten ggf. die benötigte Tensidkonzentration herab und erweitern die Eigenschaften und Anwendungsmöglichkeiten der Fluide stark.

Nanophasen-bildende-gemischt-strukturierte Amphiphile (nanophase-forming-mixed-chain structure amphiphile, NP-MCA) sind gemischt-strukturierte Amphiphile die über hydrophile und hydrophobe Molekülbereiche verfügen, die räumlich eng beieinander liegen, aber derart vermischt sind, dass sie keinen Tensid-ähnlichen Aufbau besitzen. Damit unterscheiden sie sich von Tensiden und Cotensiden, die ihre Funktion durch die gerichtete Trennung beider Bereiche erhalten (Kopf- schwanz-Struktur). Als Folge davon sind NP-MCA nicht allein zur Ausbildung von Überstrukturen fähig und reichern sich vorzugsweise nicht an der Öl-Wasser-Grenzfläche an. Zur Bildung von Nanophasenfluiden ist daher neben der Öl- bzw. Wasserphase zusätzlich noch ein Tensid nötig. NP-MCA besitzen jedoch eine signifikante Löslichkeit in der Wasserphase bzw. Ölphase und verteilen sich hierin bis zur Ausbildung eines Gleichgewichts. Die Löslichkeit des NP-MCAs in Wasser bzw. Öl beträgt in der Regel zwischen 4 und 1000 Gramm pro Liter, ggf. auch in Form seiner Salze.

Eine erfindungsgemäße NP-MCA ist eine amphiphile Substanz, die keine gerichtete hydrophilhydrophobe Tensidstruktur aufweist, alleine nicht strukturbildend, d.h. nicht Mizellen-bildend ist, deren Löslichkeit in Wasser bzw. Öl zwischen 4g und 1000g pro Liter beträgt und die sich nicht bevorzugt an der Öl-Wasser-Grenzfläche anreichert, mit der Maßgabe, dass NP-MCA nicht ausgewählt ist aus 2-Ethyl-1,3- Hexandiol, 2-Methyl-2,4-Pentandiol, 2-(n-Butyl)-2-Ethyl-1,3-Propandiol und/oder aus 1,2-Diolen.

Bei Mikroemulsionen kann im Phasendiagramm in Abhängigkeit von Temperatur und Tensidkonzentration (Fisch- oder whale-Diagramm) ein Dreieck zwischen dem X-Punkt und den Kreuzungspunkten des Grenzbereichs des einphasigen zum zweiphasigen Bereich und der parallel zu der Ordinate angelegten Tangente des beginnenden La-Gebiets aufgespannt werden. Messmethoden für die Erstellung des Tensidkonzentration-Temperatur-Phasendiagramms(Fisch- oder whale- Diagramm) sind dem Fachmann aus dem Stand der Technik bekannt. NP-MCAs führen zu einer Aufweitung des Existenzbereichs des einphasigen Bereichs, sowie zu einer Vergrößerung der Fläche dieses Dreiecks und können darüber definiert werden. Als NP-MCAs können bevorzugt alle Amphiphile Verwendung finden, die bei einer Zugabe von 4% zu einem Öl-Wasser-Tensid-System zu einer Vergrößerung der Fläche dieser Dreiecke von mindestens 5% führen, ohne dabei das Tensid-System zu verändern, bevorzugt von mindestens 10% und ganz besonders bevorzugt von mindestens 20%. In einer besonderen Ausführungsform ist die Fläche des Dreiecks in einem Bereich von 5% bis 2000% vergrößert, ohne dabei das Tensid-System zu verändern, bevorzugt von 10% bis 1000%, ganz besonders bevorzugt von 15% bis 500%.

Erfindungsgemäß ist das Reinigungsmittel daher dadurch charakterisiert, dass das NP-MCA bei einer Zugabe zu einem Öl-Wasser-Tensid-System enthaltend die Bestandteile Öl a), Tensid c) und Wasser d) von 4 Gew.-%, bezogen auf das Gesamtgewicht des Systems, dazu führt, dass sich in einem Phasendiagramm, welches den Verlauf der einphasigen und zweiphasigen und lamellaren Existenzbereiche (lamellare Phase La) des Systems in Abhängigkeit von der Tensidkonzentration und der Temperatur darstellt (Fisch-Diagramm oder "whale- diagram"), die Fläche des im Phasendiagramm enthaltenen Dreiecks, welches bestimmt ist durch die drei Eckpunkte:
i) den X-Punkt,
ii) den oberen Kreuzungspunkt des Grenzbereichs des einphasigen zum zweiphasigen Bereichs mit der parallel zur Temperaturordinate angelegten Tangente an das beginnende La-Gebiet und
iii) den unteren Kreuzungspunkt des Grenzbereichs des einphasigen zum zweiphasigen Bereichs mit der parallel zur Temperaturordinate angelegten Tangente an das beginnende La-Gebiet,
um mindestens 5% vergrößert.

Die Lage solcher Dreiecke ist in Fig. 10 verdeutlicht.

Die Methodik zur Erstellung solcher Phasendiagramme ist beispielsweise beschrieben in:
- M. Kahlweit, R. Strey, D. Haase, H. Kunieda, T. Schmeling, B. Faulhaber, M. Borkovec, H. F. Eicke, G. Busse, F. Eggers, T. Funck, H. Richmann, L. Magid, O. Soderman, P. Stilbs, J. Winkler, A. Dittrich, and W. Jahn: "How to Study Microemulsions", J. Colloid Interf. Sci., 118 (2), 436 (1987)
- Microemulsions, T. Sottmann and R. Strey in Fundamentals of Interface and Colloid Science, Volume V, edited by J. Lyklema, Academic Press (2005).

Um ein Phasendiagramm (Fischdiagramm, engl. Whale-diagram) zu erhalten werden Proben mit konstantem Verhältnis der nicht-Tensid-Komponenten und einem Tensidanteil der stufenweise ausgehend von 0 % bis zu einem gewünschten Tensidanteil (ggf. bis 100 %) erhöht wird, angesetzt. Die Stufenweite richtet sich nach den Ansprüchen an die Messgenauigkeit, wobei eine Schrittweite von 2 % meist ausreichend ist. Diese Proben werden in einem thermostatisierten Medium (bevorzugt Wasser, evtl. mit gefrierpunktsemiedrigenden Zusätzen) bei Temperaturen von - 30 °C bis 100 °C bis zur Einstellung des Phasengleichgewichts belassen und danach der Phasenzustand optisch über die Lichtstreuung beurteilt. Die Weite der Temperaturschritte ergibt sich aus der gewünschten Messgenauigkeit, wobei für technische Anwendungen meist eine Schrittweite von 1 °C ausreichend ist. Die Phasengrenzen ergeben sich aus dem Übergang von einem Phasenzustand in den nächsten, wobei der Fehler durch die Schrittweite der Temperaturmessung vorgegeben ist. Die so erhaltenen Messpunkte werden in ein Diagramm eingetragen und miteinander verbunden, wobei die Temperatur gegen den Tensidanteil aufgetragen wird. Meist genügt es, die bei einer Probe die im Messbereich existierenden Phasenzustände zu finden und über Intervallschachtelung die Phasengrenzen zu bestimmen.

Der Wert für die Phasenaufweitung der nanostrukturierten Fluid-Zusammensetzung wird dadurch bestimmt, indem ein Dreieck in das Phasendiagramm der Figur 10 dargestellt wird, in der Weise, dass eine erste Gerade a) vom X-Punkt ausgehend auf die den Phasenzustand oberhalb der mittleren Temperatur charakterisierenden Kurve (Strich über 2) gebildet wird, eine zweite Gerade b) so gebildet wird, dass sie den Öffnungswinkels von La tangential berührt und die erste Gerade a) am Ort ihres tangentialen Berührungspunktes mit der oberhalb der mittleren Temperatur charakterisierenden Kurve (Strich über 2) schneidet, und eine dritte Gerade c) auf die den Phasenzustand unterhalb der mittleren Temperatur kennzeichnende Kurve (Strich unter 2) so gelegt wird, dass sie die beiden Geraden a) und b) schneidet.

Durch Summierung der Längen der drei Geraden in Figur 10, welche einer Mikroemulsion gemäß dem Stand der Technik entspricht, ergibt einen Zahlenwert A1. Die analoge Summierung der Längen der Geraden eines Phasendiagrammes gemäß der Erfindung (Nanophasenfluid) ergibt einen Zahlenwert A2. Der Zahlenwert der durch die vorliegende Erfindung erreichten vorteilhaften Phasenaufweitung wird dadurch ermittelt, indem die Verhältniszahl aus A2/A1 gebildete wird, in dem also A2 durch A1 dividiert wird. Dieser Zahlenwert ist für die erfindungsgemäße Zusammensetzung des Nanophasenfluids größer 1,0; besonders größer 1,1; insbesondere größer 1,15; ganz besonders größer 1,2; bevorzugt größer 1,22. Dabei kann die Beeinflussung des Umfangs des Dreiecks zusätzlich oder alternativ zur Vergrößerung der Fläche des Dreiecks erfolgen.

Bevorzugte NP-MCA zeichnen sich dadurch aus, dass sie bei einer Zugabe von 4 Gew.-% bezogen auf das Gesamtgewicht des Reinigungsmittels zu einem Öl- wasser-Tensid-System enthaltend die Bestandteile a), c) und d) zu einer mindestens 5%-igen Vergrößerung des Temperaturbereichs -1.T des einphasigen Existenzbereichs des Reinigungsmittels führt, der bestimmt ist, durch die im Phasendiagramm in Abhängigkeit von Temperatur und Tensidkonzentration ermittelte Länge der zur Temperaturachse parallelen Tangente an das La-Gebiet, die begrenzt wird durch die Schnittpunkte der Tangente mit der unteren und oberen Trennlinie zwischen einphasigem und zweiphasigem Existenzbereich des Reinigungsmittels (siehe Fig. 10). Besonders bevorzugte NP-MCA führen zu einer Vergrößerung des Temperaturbereichs -1.T von 10% bis 1000%, ganz besonders bevorzugt von 20% bis 500%. Dabei kann die Beeinflussung des Temperaturbereichs -1.T zusätzlich oder alternativ zur Vergrößerung der Fläche und/oder des Umfangs des Dreiecks erfolgen.

Erfindungsgemäße NP-MCA sind ausgewählt aus Acetoacetaten der Formel II:
[Formel II]
wobei
R3 jeweils unabhängig voneinander Wasserstoff oder ein C1 bis C2 Alkyl ist und R4 ein verzweigtes oder unverzweigtes C1 bis C4 Alkyl ist;
oder aus Acetoacetaten der Formel III:
   [Formel III]
   wobei
   R5 ein C1 bis C4 Alkyl ist;

Insbesondere sind besonders bevorzugte NP-MCA ausgewählt aus folgenden Acetoacetaten: Ethylacetoacetat, iso-Propylacetoacetat, Methylacetoacetat, n- Butylacetoacetat, n-Propylacetoacetat oder tert-Butylacetoacetat.

Die genannten Acetoacetate eignen sich insbesondere zur Bereitstellung eines Schmutzentferners, Fliesenreinigers, Kosmetikentferners, Haarentfärbers, Graffitientferners, Abbeizers und/oder Nagellackentferners.

Weitere erfindungsgemäße NP-MCA sind ausgewählt aus Dionen der Formel IV
[Formel IV]
wobei
p, q, r unabhängig voneinander 0, 1 oder 2 sein können, mit der Maßgabe, dass, wenn die Summe aus p, q und r = 2 ist, die Verbindung gemäß Formel IV auch zyklisch sein kann (z.B., Cyclohexandion).

Insbesondere sind besonders bevorzugte NP-MCA ausgewählt aus folgenden Dionen: 2,3-Butandion (Diacetyl), 2,4-Pentandion (Acetylaceton), 3,4-Hexandion, 2,5-Hexandion, 2,3-Pentandion, 2,3-Hexandion, 1,4-Cyclohexandion oder 1,3-Cyclohexandion.

Die genannten Dione eignen sich insbesondere zur Bereitstellung eines Kosmetikentferners, Schmutzentferners, Nagellackentferners und/oder Graffitientferners.

Ebenfalls erfindungsgemäße NP-MCA sind ausgewählt aus Estern der Formel V
[Formel V]
wobei
R6 eine Ringbindung zu R7, CH3 oder COCH3 ist und
R7 (CH2)2-O- Ringbindung zu R6 oder (CH2)2-O-(CH2)3-CH3 ist.

Insbesondere sind besonders bevorzugte NP-MCA ausgewählt aus folgenden Estern: (2-Butoxyethyl)-acetat, Ethylencarbonat, Ethylpyruvat (2- Oxopropionsäureethylester).

Die genannten Ester eignen sich insbesondere zur Bereitstellung eines Schmutzentferners, Fliesenreinigers, Kosmetikentferners, Haarentfärbers, Graffitientferners, Abbeizers und/oder Nagellackentferners.

Weitere erfindungsgemäße NP-MCA sind ausgewählt aus Malein- bzw. Fumarsäureamiden der Formel VI

R8-HN-CO-C=C-CO-O-R9 [Formel VI]

wobei
R8 Wasserstoff, ein verzweigtes oder unverzweigtes C1- C4 Alkyl, oder ein verzweigtes oder unverzweigtes, lineares oder zyklisches C1 - C6 Alkyl ist, wobei das C1 - C6 Alkyl substituiert ist mit einer oder mehreren Gruppen ausgewählt aus
OH, NH2, COOH, CO, SO3H, OP(OH)2,
und R9 Wasserstoff oder ein verzweigtes oder unverzweigtes C1 - C4 Alkyl ist.

Insbesondere sind besonders bevorzugte NP-MCA ausgewählt aus folgenden Maleinsäureamiden und deren Methyl-, Ethyl-, Propyl- und Butylester: N- Methylmaleamid; N-Ethylmaleamid; N-(n-Propyl)-maleamid; N-(i-Propyl)- maleamid; N-(n-Butyl)-maleamid; N(i-Butylmaleamid); N-(tert.-Butylmaleamid), sowie der entsprechenden Fumarsäureamide und deren Methyl-, Ethyl-, Propyl- und Butylester.

Weitere erfindungsgemäße NP-MCA sind ausgewählt aus: Diacetanalkohol (2- Methyl-2-pentanol-4-on).

Ganz besonders bevorzugt sind die folgenden NP-MCA, welche ausgewählt sind aus der Gruppe bestehend aus Ethylacetoacetat; i-Propylacetoacetat; Methylacetoacetat; Methylisobutyrylacetat (Methyl-(4-methyl-3-oxopentanoat)); n- Butylacetoacetat; n-Propylacetoacetat; tert-Butylacetoacetat; Allylacetoacetat; Maleinsäureamid (Maleamische Säure, Maleamid), folgende Maleamide und deren Methyl-, Ethyl-, Propyl- und Butylester: N-Methylmaleamid; N- Ethylmaleamid; N-(n-Propyl)-maleamid; N-(i-Propyl)-maleamid; N-(n-Butyl)-maleamid; N(i-Butylmaleamid); N-(tert.-Butylmaleamid); sowie der entsprechenden Fumarsäureamide und deren Methyl-, Ethyl-, Propyl- und Butylester;; Diacetonalkohol (4-Hydroxy-4-methylpentan-2-on); (2-Butoxyethyl)-acetat; 1,3- Cyclohexandion; 1,4-Cyclohexandion; 2,3-Hexandion; 2,3-Pentandion; 2,5- Hexandion; 3,4-Hexandion; Acetylaceton (2,4-Pentandion, ACAC); Diacetyl (2,3- Butandion); Ethylencarbonat; Ethylpyruvat (2-Oxopropionsäureethylester);
einzeln oder als Gemisch.

Bevorzugt ist das NP-MCA im erfindungsgemäßen Reinigungsmittel von 1 - 80 Gew.-% enthalten bezogen auf das Gesamtgewicht des Reinigungsmittels, besonders bevorzugt von 2 - 25 Gew.-%, ganz besonders bevorzugt von 10 - 24 Gew.-%.

Unter der mindestens einen wasserunlöslichen Substanz mit einer Löslichkeit in Wasser von weniger als 4 g pro Liter werden für die Zwecke der vorliegenden Erfindung Öle verstanden. Mit Öl werden dabei alle hydrophoben Stoffe bezeichnet, die sich nicht mit Wasser oder einer wasserähnlichen Flüssigkeiten homogen mischen und eine separate Phase bilden. Da einige Öle sich noch zu einem Großteil in Wasser lösen, wird hier zusätzlich eine Wasserlöslichkeit von kleiner als 4 Gramm pro Liter definiert. Bevorzugt handelt es sich bei den wasserunlöslichen Substanzen um solche mit einer Wasserlöslichkeit kleiner 2 g pro Liter. Hierzu zählen z. B. Alkane (Benzine) und Cycloalkane (vorzugsweise Cyclohexan). Auch Aromaten wie Toluol, Xylole oder andere Alkylbenzole sowie Naphthaline kommen in Frage. Bevorzugt sind langkettige Alkansäureester, wie fette Öle und Fettsäurealkylester oder Fettalkoholether. Auch Benzylacetat gehört erfindungsgemäß zu den eingesetzten wasserunlöslichen Substanzen. Aber auch Terpene, z. B. monocyclische Monoterpene mit Cyclohexangerüst, können Verwendung finden. Besonders bevorzugt sind hier Terpene aus Zitrusfrüchten, wie Citronen- und/oder Orangenterpene bzw. das darin enthaltene Limonen. Die wasserunlöslichen Substanzen sind vorzugsweise von 1 - 90 Gew.-% im Reinigungsmittel enthalten, besonders bevorzugt von 1,5 - 30 Gew.-% bezogen auf das Gesamtgewicht des Reinigungsmittel.

Als weitere amphiphile Substanzen werden erfindungsgemäß Cotenside mit hydrophil-lipophilen Molekülanteilen wie z. B. dien- und i-Isomere von Butanol, Pentanol, Hexanol, Heptanol, Octanol, Nonanol, Decanol, Undecanol und Dodecanol, verwendet.

Bevorzugt sind auch Cycloalkanole, wie Cyclohexanol oder besonders bevorzugt Phenylalkohole wie Phenylmethanol (Benzylalkohol), 2-Phenylethanol und 3- Phenyl-1-propanol.

Ebenso können kurzkettige Fettsäuren, wie Hexan-, Heptan-, Octansäure und deren Alkali- oder Ammoniumsalze Verwendung finden. Besonders bevorzugt sind deren Salze von Ethanolaminen.

Cotenside sind vorzugsweise von 2 bis 45 Gew.-% in der erfindungsgemäßen Zusammensetzung enthalten, bezogen auf das Gesamtgewicht des Reinigungsmittels, besonders bevorzugt von 2 bis 40 Gew.-%.

Besonders bevorzugt weist die weitere amphiphile Substanz eine Wasserlöslichkeit von 2 g bis 128 g pro Liter auf und ist ausgewählt aus der Gruppe umfassend C4-C12-Alkohole, Cycloalkanole, Phenylalkohole, kurzkettige Fettsäuren oder deren Alkali- oder Ammoniumsalze.

Das Reinigungsmittel umfasst weiterhin anionische, kationische, amphotere und/oder nichtionische Tenside. In der folgenden Aufstellung werden einige bevorzugt geeignete Tenside genannt.

Als anionische Tenside können z. B. Alkali- oder Ammonium-Salze von langkettigen Fettsäuren, Alkyl(benzol)sulfonate, Paraffinsulfonate, Bis(2- ethylhexyl) sulfosuccinat, Alkylsulfate, wie v.a. Natriumdodecylsulfat und für spezielle Anwendungen, bei denen es z.B. auf einen Korrosionsschutz ankommt, teilweise auch Alkylphosphate (z.B. Phospholan<^{®}> PE 65, Akzo Nobel) eingesetzt werden. Als nichtionische Tenside können Polyalkylenoxidmodifizierte Fettalkohole, wie z.B. Berok^{®}>-Typen (Akzo-Nobel) und Hoesch T-Typen (Julius Hoesch), sowie auch entsprechende Octylphenole (Triton-Typen) oder Nonylphenole (sofern letztere nicht in großen Mengen in die Umwelt freigesetzt werden). Ein besonderes Einsatzgebiet ermöglichen die Heptamethyltrisiloxane (z.B. Silwek^{®}>-Typen, GE Silicones), als Mittel zur starken Erhöhung der Spreiteigenschaften der Flüssigkeiten bzw. zur deutlichen Absenkung der Grenzflächenspannung.

Als kationische Tenside können z.B. Kokosbis (2-hydroxyethyl-) methylammoniumchlorid oder Polyoxyethylen-modifiziertes Talkmethylammoniumchlorid Verwendung finden. Daneben ist auch der Einsatz diverser amphoterer Tenside möglich. Soll ein weiter ph-Bereich abgedeckt werden, so hat sich das Kokosdimethylaminoxid (Aromox^{®} MCD, Akzo-Nobel) als geeignet herausgestellt.

Die Tenside sind im Reinigungsmittel vorzugsweise von 9 bis 16 Gew.-% enthalten, bezogen auf das Gesamtgewicht des Reinigungsmittels.

Die erfindungsgemäßen Reinigungsmittel können gegebenenfalls mit Hilfsstoffen versetzt werden, die die Anwendung erleichtern bzw. verbessern. Hierbei geht es
z. B. um eine Verdickung zur Erleichterung des Auftrags des Reinigungsmittels auf den Untergrund, um eine Erniedrigung der Entzündlichkeit, eine geruchlichen Verbesserung, eine Wirkungsverstärkung, eine Kostenreduzierung der Wirkstoffe oder um einen Korrosionsschutz. Die Summe der Hilfsstoffe macht vorzugsweise nicht mehr als 20 Gew.-% der Formulierung aus, die Menge eines einzelnen Hilfsstoffes beträgt oft nicht mehr als 10 Gew.-%.
a) Verdickung: Bei den meisten Flächen eines Anwendungsfalls handelt es sich um senkrechte Wände, von denen niedrigviskose Flüssigkeiten vom Schadensort eines Graffito nach unten zu schnell ablaufen. Eine Verdickung der Flüssigkeiten ist also günstig, um das Ablaufen zu verzögern oder gar zu verhindern. Um die aus den Versuchen resultierenden niedrigviskosen Flüssigkeiten zu verdicken, können handelsübliche Verdickungsmittel eingesetzt werden. Besonders bevorzugt ist pyrogene Kieselsäure, wie z.B. Aerosik^{®}> 380.
b) Flammschutz: Da der Graffitientferner einige leichtflüchtige Stoffe enthalten kann, ist ein Flammschutzmittel gegebenenfalls sinnvoll. Durch Zusatz von z.B. Triethylphosphat (TEP) oder Trioctylphosphaten, wie dem Tris(2- ethylhexyl)phosphat (TEHP), kann das Mittel selbsterlöschend eingestellt werden.
c) Geruchliche Verbesserung: Falls für den Endverbraucher die Formulierung geruchlich verbessert werden soll, so kann dies mit einer Reihe üblicher Geruchsstoffe vorgenommen werden. Als Beispiele seien Ester, wie Methylbutanoat (Ananas), Ethylmethanoat (Himbeere), Pentylethanoat (Banane), Pentylpentanoat (Apfel), Pentylbutanoat (Aprikose) Octylethanoat (Orange) genannt. Letzteres kann z. B. auch gut mit Orangenterpenen bzw. D-Limonen kombiniert werden, was ein Beispiel für einen lipophilen Wirkstoff in der erfindungsgemäßen Flüssigkeit darstellen kann. Auch andere Geruchsstoffe finden sich im Bereich der Terpene, wie z. B. Geraniol.

In einer bevorzugten Ausführungsform weist das Reinigungsmittel die folgende Zusammensetzung auf:
1-90 Gew.-% wasserunlösliche Substanz a) 1-80 Gew.-% NP-MCA b)
2-45 Gew.-% Tensid c), einschließlich ggf. weitere Cotenside 1-90 Gew.-% Wasser
ggf. Hilfsstoffe bis max. 10 Gew.-%, wobei sich die Prozentangaben jeweils auf das Gesamtgewicht des Reinigungsmittels beziehen.

Die erfindungsgemäße Zusammensetzung, die eine Flüssigkeit darstellt, bestehend aus mindestens vier aber meist aus mehr Komponenten, löst überraschenderweise Lacke und Beschichtungen deutlich effektiver, sauberer und teilweise schneller von verschiedenen Untergründen ab, als
b) Flammschutz: Da der Graffitientferner einige leichtflüchtige Stoffe enthalten kann, ist ein Flammschutzmittel gegebenenfalls sinnvoll. Durch Zusatz von z.B. Triethylphosphat (TEP) oder. Trioctylphosphaten, wie dem Tris(2-ethyl- hexyl)phosphat (TEHP), kann das Mittel selbsterlöschend eingestellt werden.
c) Geruchliche Verbesserung: Falls für den Endverbraucher die Formulierung geruchlich verbessert werden soll, so kann dies mit einer Reihe üblicher Geruchsstoffe vorgenommen werden. Als Beispiele seien Ester, wie Methylbutanoat (Ananas), Ethylmethanoat (Himbeere), Peritylethanoat (Banane), Pentylpentanoat (Apfel), Pentylbutanoat (Aprikose) Octylethanoat (Orange) genannt. Letzteres kann z. B. auch gut mit Orangenterpenen bzw. D- Limonen kombiniert werden, was ein Beispiel für einen lipophilen Wirkstoff in der erfindungsgemäßen Flüssigkeit darstellen kann. Auch andere Geruchsstoffe finden sich im Bereich der Terpene, wie z.B. Geraniol.

In einer bevorzugten Ausführungsform enthält das Reinigungsmittel: 1-90% wasserunlösliche Substanz a)
1-80% NP-MCA b)
2-45% Tensid c), einschließlich ggf. weitere amphiphile Substanz 1-90% Wasser
ggf. Hilfsstoffe bis max. 10%,
wobei sich die Prozentangaben jeweils auf das Gesamtgewicht des Reinigungsmittels beziehen.

Die erfindungsgemäße Zusammensetzung, die eine Flüssigkeit darstellt, bestehend aus mindestens vier aber meist aus mehr Komponenten, löst überraschenderweise Lacke und Beschichtungen deutlich effektiver, sauberer und teilweise schneller von verschiedenen Untergründen ab, als
a) konventionelle Graffitientferner (vgl. Fig. 1), Abbeizer oder Nagellackentferner auf der üblichen Lösungsmittel-Basis bzw. als generell Lösungsmittel, selbst wenn diese als gut solvatisierend bekannt sind
b) die Einzelkomponenten für sich (vgl. Fig. 2)
c) Gemische der Lösungsmittel-Komponenten ohne Wasser und/oder ohne Tenside
d) konventionelle Graffitientferner auf bekannter Emulsions-Basis bzw. die aus den Lösungsmittel-Komponenten hergestellten Emulsionen
e) Gemische von Wasser und Tensiden.

Darüber hinaus lösten diese _Flüssigkeiten die Graffiti-Farben mindestens ebenso gut vom Untergrund, wie die wirkungsvollsten Lösungsmittel der darüber hinaus getesteten Lösungsmittel, also namentlich z.B. Tetrahydrofuran, 1-Methyl-2- pyrrolidon, 1-Methoxy-2-propanol, Butyrolacton oder Dichlormethan.

Die erfindungsgemäßen Flüssigkeiten zeigten im Hinblick auf ihr Löseverhalten noch weitere überraschende Effekte:
a) der primäre Reinigungseffekt bestand nicht a priori in einem physikalischen Lösen des Lackes, da kein Lackbindemittel von den Flüssigkeiten molekulardispers gelöst wird, sondern in einem
b) starken Anquellen des Lacks, teilweise in wellenartiger Form, teilweise mit mikroskopisch kleinen Löchern in der Lackschicht und einem anschließendem Zerteilen (Fragmentieren) der Lackschicht in ca. 100 µm bis 2 mm große Lackstücke und
c) einem Unterwandern großer Bereiche der Lackschicht mit anschließendem Abheben der Schicht.
d) Alle behandelten, verschiedenartig strukturierten Untergründe zeigten praktisch keine Farbschatten, wenn der Lack mit feuchten Reinigungswerkzeugen oder Niederdruck-Spritzen (3 - 20 bar) behandelt wurde, da die Flüssigkeiten die Lacke nicht molekulardispers lösten, somit keine feinteiligen Pigmentpartikel freisetzten und in Verbindung mit Wasser als Waschlauge fungierten.

Nach einer Einwirkzeit von nur wenigen Sekunden bis Minuten konnten Lacke und Beschichtungen z.B. durch einen feuchten Schwamm, Bürste oder Pinsel vollständig abgelöst werden, wobei in Verbindung mit Wasser aus der vorher transparenten Flüssigkeit eine Emulsion entstand, die zwar ein deutlich geringeres Ablösevermögen, dafür aber eine ausgeprägte Waschwirkung besaß und die Farbpartikel bzw. Bindemittelreste- vom Untergrund entfernte. Aufgrund dieser Waschwirkung entstand kein feiner farblicher Gradient ("Schatten"), den übliche Lösungsmittel(-Gemische) normalerweise vom Anwendungsort hin zu unbehandelten Stellen auf dem Untergrund hinterlassen und der aus verdünnten Bindemitteln und Pigmenten besteht.

Der Reinigungseffekt der erfindungsgemäßen Flüssigkeiten besteht also in zwei Stufen:
a) in unverdünnter Form: Anquellen oder Anläsen von Bindemittel-Systemen der Graffiti-Farbe meist in Verbindung mit einem Fragmentieren des Lackes
   - im Falle des Anquellens auch eine Unterwanderung der Farbschicht bis hin zum Untergrund
b) zusammen mit geringen Mengen Wasser, wie sie z.B. in einem feuchten Schwamm, Bürste oder Pinsel vorkommen: Bildung einer Waschlauge und Abheben der angequollenen und/oder aufgelösten Lackpartikel vom Untergrund.

Im Gegensatz zu bisherigen Lack-Entfernern auf üblicher Lösungsmittel-Basis ergibt sich auf den erfindungsgemäß behandelten Flächen also kein Farbgradient, d. h. ein Verschmieren der aufgelösten Graffitifarbe - weder zu den Rändern der Beschichtungen hin, noch in den Untergrund hinein. Die Fotographie in Fig. 1 von einem behandelten groben Rauputz-Untergrund zeigt diese Wirkung im Vergleich zu einem konventionellen Graffitientferner auf Basis von 1-Methoxy-2-propanol. Die Fotographie in Fig. 2 zeigt dieselbe Wirkung beim Entfernen von Nagellack

Im Gegensatz zu bisherigen Graffitientfernern auf üblicher Lösungsmittel-Basis zeigen die erfindungsgemäßen Systeme ein sehr breites Wirkungsspektrum auf das Ablösen der Farben bzw. Beschichtungen, ohne die Einschränkungen auf bestimmte Bindemittel-Typen, wie es häufig bei der Verwendung· von Lösungsmitteln der Fall ist. Gegenüber Dispersionsfarben, die Farbüberzüge durch Koaleszens feinster, hochpolymerer Partikel erzeugen und häufig als Fassadenfarben eingesetzt werden, zeigt das erfindungsgemäße Graffitientferner-System eine Selektivität, d. h. hochwertige Fassadenfarben werden innerhalb der Einwirkungszeit nicht abgelöst. Im Fall des Abbeizers ist diese Selektivität nicht erforderlich, da hier auch Untergrund-Beschichtungen mit abgehoben werden müssen.

Im Gegensatz zu Lackentfernern auf üblicher Lösungsmittel-Basis zeigen die erfindungsgemäßen Systeme ein sehr schnelles Anquellen und Ablösen der Farben; je nach Alter, Untergrund und Farbe üblicherweise innerhalb von 1O Sekunden bis etwa 30 Minuten (vorzugsweise 20 Minuten) beim Graffitientfemer, 20 Minuten bis 3

Stunden beim Abbeizer und 3 bis 30 Sekunden (vorzugsweise 3 bis 20 Sekunden) beim Nagellackentferner.

Die Erfindung bezieht sich also auch auf die Verwendung erfindungsgemäßer Reinigungsmittel als Graffitientferner, Abbeizer oder Nagellackentferner.

Gegenstand der Erfindung ist auch ein Verfahren zur Entfernung unerwünschter Farben und Lacke von Oberflächen. Das erfindungsgemäße Verfahren zur Entfernung unerwünschter Farben und Lacke von Oberflächen zeichnet sich dadurch aus, dass das erfindungsgemäße Reinigungsmittel auf die unerwünschte Farbe oder den Lack aufgebracht wird, einwirkt und anschließe d die Farbe oder der Lack mit Wasser entfernt wird, wobei die Einwirkzeit beim Graffitientferner von etwa 10 Sekunden bis etwa 30 Minuten beträgt, beim Abbeizer von etwa 20 Minuten bis etwa 3 Stunden und beim Nagellackentfernen von etwa 3 bis etwa 30 Sekunden.

Die Erfindung bezieht sich des Weiteren auf die Verwendung erfindungsgemäßer Reinigungsmittel als Schmutzentferner, Fliesenreiniger, Kosmetikentferner oder Haarentfärber ohne oxidative Bleiche. Unter Schmutz wird dabei das Vorhandensein von mindestens einer Komponente ausgewählt aus Ruß, Fett, Öl, Silikon, Feinstaub, Harz und/oder Mischungen enthaltend einen oder mehrere dieser Bestandteile verstanden. Unter Kosmetik werden Mittel zur Körper- und Schönheitspflege verstanden, insbesondere solche Mittel, die aus die Haut und/oder Hautanhangsgebilde, wie beispielsweise Harre oder Nägel, aufgetragen werden. Unter Haar wird sowohl Kunsthaar als auch echtes Naturhaar verstanden. Unter Haarfarben werden Mittel verstanden, die Haare färben ohne sie oxidativ zu bleichen.

Hierin beschrieben ist auch ein Verfahren zur Entfernung von Schmutz (beispielsweise Ruße, Fette, Öle, Silikone, Feinstäube, Harze und Mischungen enthaltend einen oder mehrere dieser Bestandteile) von Oberflächen wie beispielsweise Keramik-, Fliesen- oder Kunststoffoberflächen, von Kosmetik oder zur Haarentfärbung, dadurch gekennzeichnet, dass ein Reinigungsmittel, wie oben beschrieben, auf den Schmutz, die zu entfernende Kosmetik oder die zu entfärbenden Haare aufgebracht wird, einwirkt und anschließend der Schmutz, das Mittel oder die Farbe mit Wasser entfernt wird, wobei die Einwirkzeit beim Schmutzentferner von etwa 10 Sekunden bis etwa 3 Stunden beträgt, beim Kosmetikentferner von etwa 10 Sekunden bis etwa 30 Minuten und beim Haarentfärber von etwa 2 Minuten bis etwa 24 Stunden.

Des Weiteren wird ein Verfahren zur Herstellung des oben beschriebenen Reinigungsmittels dargestellt. Das Verfahren zur Herstellung eines Reinigungsmittels zeichnet sich dadurch aus, dass Wasser oder ein Lösungsmittel mit Hydroxy-Funktionalität vorgelegt und ein anionisches, kationisches, amphoteres und/oder nicht ionisches Tensid bei 10 bis 90 °C unter Rühren darin aufgelöst wird, wasserunlösliche Substanz(en) parallel oder nach Tensid-Zugabe zugesetzt werden und dann die entstandene Emulsion durch die Zugabe eines Cotensids und eines NP-MCAs in ein nicht koaleszierendes, thermodynamisch stabiles Nanophasensystem überführt wird und am Ende des Mischungsvorgangs gegebenenfalls Hilfsstoffe zugefügt werden.

Das Reinigungsmittel wird hergestellt, indem in einem geeigneten Gefäß zunächst Wasser bzw. das Lösungsmittel mit Hydroxy-Funktionalität vorgelegt und dann das Tensid unter Rühren aufgelöst wird. Hierbei ist zu beachten, dass einige Tenside bereits Wasser in Lieferform enthalten können, so dass die in der Rezeptur vorausberechnete Wassermenge ggf. korrigiert werden muss. Beim Auflösen des Tensids muss darauf geachtet werden, dass der Lufteintrag in die Lösung so gering wie möglich gehalten wird, um ein übermäßiges Schäumen zu vermeiden. Für die großtechnische Realisierung gibt es bereits viele Variationen an Rührwerken und Rührer, um ein Schäumen weitgehend zu vermeiden. Die Rührgeschwindigkeit sollte bei Verwendung von Propellerrührern und idealen Verhältnissen von Rührerdurchmesser und Gebindedurchmesser üblicherweise 200 Umdrehungen pro Minute nicht überschreiten. Weiterhin muss darauf geachtet werden, dass einige (konzentrierte) Tenside bei Zugabe von Wasser Gele bilden können, die ein Rühren und eine weitere Verteilung erschweren können. In solchen Fällen müssen ggf. die wasserunlöslichen Substanzen (Ölphase) zuerst oder parallel zur Tensid-Zugabe zugegeben werden. Ein Schäumen kann auch durch die nachfolgende Zugabe der Ölphase verhindert werden, da diese oft eine gewisse Entschäumerwirkung besitzen. Nach Zugabe der Ölphase ist eine milchig-trübe Emulsion entstanden, die durch die Zugabe des Cotensids mit Tensidstruktur (z.B. Alkanol), spätestens aber nach Zugabe des Amphiphils nach Anspruch 1b (z.B. der Acetoacetat-Verbindung) aufklart und schließlich in ein nicht koaleszierendes, thermodynamisch stabiles Nanophasensystem übergeht. Am Ende können noch Zusatzstoffe, wie z.B. flammhemmende Mittel (z.B. Triethylphosphat), Verdickungsmittel (z.B. Aerosile) und/oder andere Hilfsmittel zugefügt werden.

Des weiteren wird ein Verfahren zur Herstellung eines erfindungsgemäßen Reinigungsmittels beschrieben, dadurch gekennzeichnet, dass Wasser oder ein Lösungsmittel mit Hydroxy-Funktionalität vorgelegt und ein anionisches, kationisches, amphoteres und/oder nicht ionisches Tensid bei 10 bis 90 °C unter Rühren darin aufgelöst wird, wasserunlösliche Substanz(en) parallel oder nach Tensid-Zugabe zugesetzt werden und dann die entstandene Emulsion durch die Zugabe eines Cotensids und eines NP-MCAs in ein nicht koaleszierendes, thermodynamisch stabiles Nanophasensystem überführt wird und am Ende des Mischungsvorgangs gegebenenfalls Hilfsstoffe zugefügt werden.
Es zeigen Fig: 1:
   Vergleich des erfindungsgemäßen Graffitientferners nach Beispiel 2 mit einem konventionellen Graffitientferner auf Basis von 1-Methoxy-2-propanol (Baufan RICO Graffiti-Killer)
   Fotografische Aufnahme einer mit Graffiti beschädigten Wand mit grobem Rauputz (Alter des Graffito mindestens 1 Jahr). Gleichzeitige und gleichlange Einwirkung der Graffitientferner, nach 5 Minuten Abwischen der Entfemer mit einem Schwamm und anschließendes Abspülen der Wand mit einem Niederdruck- Wasserstrahl (< 3 bar):
      a) Die erfindungsgemäße Formulierung zeigt keine Farbschatten, keine Beeinträchtigung der ursprünglichen Fassadenfarbe (weiß) und keinen Farbgradienten an den Rändern, sondern eine scharfe Abgrenzung zur unbehandelten Fläche.
      b) Der konventionelle Graffitientferner auf Basis von 1-Methoxy-2-propanol zeigt dagegen eine schlechtere Farbentfernung mit unscharfen Rändern vom Wirkungsort zur unbehandelten Fläche, was durch ein Verschmieren der Graffiti- Farbe verursacht wird. Zudem ist die durch das Mittel verdünnte Farbe teilweise von den Poren des Putzes aufgenommen worden, was Farbschatten erzeugt, die nachträglich nur schwer zu entfernen sind.
Fig. 2:
   Wirksamkeit des konventionellen Nagellackentferners essence^{®} mit Ethylacetat als Lacklösungsmittel, des erfindungsgemäßen Nagellackentferners des Beispiels 5 und von Einzelkomponenten
   a) gute Lackentfernung, Verschmierung zum Rand hin
   b) gute Lackentfernung des Nanophasenfluids, scharfe Ränder mit gequollenen Bereichen
   c) nahezu kein Ablösen, kein Anquellen
   d) kaum Lackentfernung
   e) Lackentfernung mit starkem Verschmieren zum Rand hin
   Die Einzelkomponenten c) und d) des Nanophasenfluids b) zeigen für sich keine Wirksamkeit auf das Ablösen des Lackes; Komponente e) ist ein schlechtes Lösungsmittel.
Fig. 3:
   Wirksamkeit erfindungsgemäßer Reinigungsmittel als Schmutzentferner mit a) Zustand vor der Reinigung und b) Zustand nach der Reinigung. Unter 1 wurde ein handelsübliches Reinigungsmittel verwendet und unter 2 ein erfindungsgemäßes Reinigungsmittel mit Nanophasenstrukturierung.
Fig. 4:
   Wirksamkeit erfindungsgemäßer Reinigungsmittel als Kosmetikentferner mit a) Zustand vor der Reinigung und b) Zustand nach der Reinigung. Unter 1 wurde ein handelsüblicher Kosmetikentferner verwendet und unter 2 ein erfindungsgemäßes Reinigungsmittel mit Nanophasenstrukturierung. Die Zahlen ganz unten in b) geben die Reinigungszyklen an, d.h. wie viel Mal wurde mit einem Watte-Pad, der mit dem entsprechenden Reinigungsmittel getränkt war, mit leichtem Druck und nur in eine Richtung über die Stelle gewischt.
Fig. 5:
   Wirksamkeit erfindungsgemäßer Reinigungsmittel als Haarentfärber mit a) Zustand nach der Reinigung und b) Zustand vor der Reinigung.
Fig. 6:
   Streuung eines Laserstrahls zum Nachweis der Nanostrukturierung in flüssigen Systemen mit a) Ethylacetoacetat, b) Aceton, c) erfindungsgemäßes Reinigungsmittel graffitiCRACK, d) erfindungsgemäßes Reinigungsmittel LisoCLEAR, e) erfindungsgemäßes Reinigungsmittel V1113.
Fig. 7:
   Wirksamkeit erfindungsgemäßer Reinigungsmittel bei der Lackentfernung im Vergleich zu nicht Nanophasen bildenden Reinigungsmitteln, wobei die Ergebnisse nach der Reinugung gezeigt sind für a) Ethylacetoacetat, b) V141 (keine Nanophase), c) erfindungsgemäßes Reinigungsmittel NP1, d) erfindungsgemäßes Reinigungsmittel NP2, e) erfindungsgemäßes Reinigungsmittel erfindungsgemäßes Reinigungsmittel NP3, f) V142 (keine Nanophase).
Fig. 8:
   Wirksamkeit erfindungsgemäßer Reinigungsmittel bei der Fliesenreinigung im Vergleich zu nicht Nanophasen bildenden Reinigungsmitteln, wobei jeweils in a) der Zustand vor der Reinigung und in b) der Zustand nach Reinigung dargestellt ist. Unter 1) sind die Ergebnisse für eine Mikroemulsion gezeigt, unter 2) die Ergebnisse für ein Emulsionssystem und unter 3) die Ergebnisse eines erfindungsgemäßen Nanophasensystems.
Fig. 9:
   In Fig. 9 ist mittels einer Gefrierbruch-elektronenmikroskopischen Aufnahme (freeze- fracture electron microscopy) die Nanostrukturierung des Fluids NP2 zu erkennen. Bei den kleineren kugelförmigen Strukturen (Pfeil) handelt es sich um ca. 20 - 50 nm große Mizellen der Wasserphase, die innerhalb einer gering strukturierten Ölphase verteilt sind
Fig.10:
   Phasendiagramm (Fisch-Diagramm oder whale-Diagramm), welches den Verlauf der einphasigen und zweiphasigen und lammellaren Existenzbereiche eines Reinigungsmittels in Abhängigkeit von der Tensidkonzentration und der Temperatur darstellt. In a) ist eine Zusammensetzung als Mikroemulsion gezeigt, in b) die gleiche Zusammensetzung zusätzlich enthaltend 4% NP-MCA als Nanophasenfluid. Dargestellt ist der Temperaturbereich, AT, des einphasigen Existenzbereichs des Reinigungsmittels, wobei AT bestimmt ist, durch die im Fisch-Diagramm ermittelte Länge der zur Temperaturachse parallelen Tangente an das La-Gebiet, die begrenzt wird durch die Schnittpunkte der Tangente mit der unteren und oberen Trennlinie zwischen einphasigem und zweiphasigem Existenzbereich des Reinigungsmittels. Wie aus Fig. 10 ersichtlich führt das Vorhandensein von NP-MCA zu einer Vergrößerung des Temperaturbereichs AT.

Folgende Ausführungsbeispiele sollen die Erfindung näher erläutern, ohne sie darauf einzuschränken.

### Ausführungsbeispiele

### Beispiel 1: Graffitientferner

| **Stoff** | **Masseprozent [%]** |
|---|---|
| Wasser demineralisiert | 20,00 |
| Triethylphosphat | 4,50 |
| Ethylacetoacetat | 17,20 |
| Orangenterpene | 30,00 |
| Natriumlaurylsulfat | 12,30 |
| 1-Hexanol | 16,00 |
| | 100,00 |

Der Graffitientferner aus Beispiel 1 wurde hergestellt, indem in einem geeigneten Gefäß zunächst Wasser vorgelegt und darin das Tensid (Natriumlaurylsulfat) unter Rühren aufgelöst wurde. Beim Auflösen des Tensids sollte der Lufteintrag in die Lösung so gering wie möglich gehalten werden. Ein Schäumen kann durch die nachfolgende Zugabe der Orangenterpene verhindert werden, da diese eine gewisse Entschäumerwirkung besitzen. Nach diesem Schritt ist eine milchig-trübe Emulsion entstanden, die durch die Zugabe von 1-Hexanol und Ethylacetoacetat klar wird und schließlich in ein völlig transparentes Nanophasensystem übergeht. Am Ende wird das Triethylphosphat zugefügt.

### Beispiel 2: Graffitientferner

| **Stoff** | **Masseprozent [%]** |
|---|---|
| Wasser demineralisiert | 24,50 |
| Triethylphosphat | 5,00 |
| Ethylacetoacetat | 14,50 |
| Orangenterpene | 27,50 |
| Natriumolefinsulfonat (Hansanyl OS) | 12,50 |
| 1-Hexanol | 16,00 |
| | 100,00 |

Der Graffitientferner aus Beispiel 2 wurde verfahrenstechnisch analog Beispiel 1 hergestellt.

### Beispiel 3: Graffitientferner

| **Stoff** | **Masseprozent [%]** |
|---|---|
| Wasser demineralisiert | 32,00 |
| Triethylphosphat | 4,50 |
| Ethylacetoacetat | 11,50 |
| n-Butylacetat | 8,00 |
| Orangenterpene | 12,00 |
| Benzylacetat | 8,00 |
| Natriumdodecylsulfat | 11,00 |
| 1-Hexanol | 13,00 |
| | 100,00 |

Der Graffitientferner aus Beispiel 3 wurde verfahrenstechnisch analog Beispiel 1 hergestellt.

### Beispiel 4: Abbeizer

| **Stoff** | **Masseprozent [%]** |
|---|---|
| Wasser demineralisiert | 30,77 |
| Triethylphosphat | 4,32 |
| Ethylacetoacetat | 11,06 |
| Dibasen Ester (DuPont) | 7,69 |
| Benzylacetat | 7,69 |
| Orangenterpene | 15,39 |
| Hoesch NAS (als 100%-ige Substanz) | 10,58 |
| 1-Hexanol | 12,50 |

Der Abbeizer aus Beispiel 4 wurde verfahrenstechnisch analog Beispiel 1 hergestellt.

### Beispiel 5: Nagellackentferner

| **Stoff** | **Masseprozent [%]** |
|---|---|
| Wasser demineralisiert | 43,49 |
| Ethylacetoacetat | 22,26 |
| 2-Phenylethanol | 14,84 |
| Ätherisches Öl Orangen (Duftstoff) | 2,45 |
| Natriumlaurylsulfat | 16,96 |
| | 100,00 |

Der Nagellackentferner aus Beispiel 5 wurde hergestellt, indem das Tensid (Natriumlaurylsulfat) unter leichtem Rühren bei Rührgeschwindigkeiten von100 Umdrehungen pro Minute in eine Mischung aus Wasser, Ethylacetoacetat und 2-Phenylethanol eingerieselt wurde. Nach Bildung des Nanophasensystems wurde der Duftstoff hinzugefügt.

Die folgende Übersicht zeigt die Wirkung der erfindungsgemäßen Graffitientferner im Vergleich zu konventionellen Graffitientfernern bzw. Lösungsmitteln

**Tabelle 1: Wirkung der in den Beispielen 1 und 3 hergestellten Graffitientferner der Erfindung innerhalb von 5 Minuten Einwirkzeit.**

| | | **Beispiel** | **Beispiel** | **Graffitientferner auf Basis** | |
|---|---|---|---|---|---|
| **Farbe** | **Untergrund** | **1** | **3** | **1-Methoxy-2-propanol** | **2-Butanol** |
| schwarz | Feinstein rau, mikroporös | vollständig | vollständig | Farbschatten | Kaum Entfernung |
| | | | | | |

| | | **Beispiel** | **Beispiel** | **Graffitientferner auf Basis** | |
|---|---|---|---|---|---|
| **Farbe** | **Untergrund** | **1** | **3** | **1-Methoxy-2-propanol** | **2-Butanol** |
| schwarz | | vollständig | vollständig | keine Entfernung in Poren | Keine Entrfernung Poren |
| schwarz | | vollständig | Vollständig | vollständig | Kaum Entfernung |
| schwarz | | vollständig | Vollständig | Farbgradient | Zu ca. 80% Entfernung |
| schwarz | | vollständig | Vollständig | vollständig | Kaum Entfernung, Schmieren |
| rot | | vollständig | Vollständig | Farbschatten | Kaum Entfernung |
| dunkelrot | Rauhputz weiss gestrichen | vollständig | Vollständig | Farbschatten | Kein Ergebnis vorhanden |
| gelb | | vollständig | Vollständig | fast vollständig | |
| dunkelgrün | | vollständig | Vollständig | | |
| | | vollständig | Vollständig | | |
| türkisgrün | | vollständig | Vollständig | | |
| dunkelblau | | vollständig | Vollständig | | |
| blau | | vollständig | Vollständig | | |
| blau | | vollständig | Vollständig | | |
| gold metallic | | vollständig | Vollständig | | Fast vollständig |
| silber metallic | | vollständig | Vollständig | | Fast vollständig |

**Tabelle 2: Wirkung des erfindungsgemäßen Nagellackentferners nach Beispiel 5: nach einmaligem Auftrag des Entfemers mit Watte-Pads und anschließendem Abwaschen der Nägel mit Wasser**

| **Nagellack** | | **Beispiel** | | **"essence pocket beauty"** |
|---|---|---|---|---|
| **Farbe** | **Marke** | **5** | **Aceton** | **Ethylacetat** |
| **Nagellack** | | **Beispiel** | | **"essence pocket beauty"** |
| | | | | |
| **Farbe** | **Marke** | **5** | **Aceton** | **Ethylacetat** |
| dunkelviolett | BIG BLUE | nach 3 Sekunden Ablösen | vollständig, jedoch mit Farbschmieren an Haut | nach 3 Sekunden auflösen, Farbschmieren an Haut |
| braun-gold | BIG BLUE | nach 3 Sekunden Ablösen, vollständig nach ca. 10 Sekunden | keine vollständige Entfernung des Lacks von Haut und Nagel | nach 3 Sekunden auflösen, Farbschmieren an Haut |
| rosa | essence cosnova | Nach ca. 10 Sekunden vollständig | vollständig | vollständig |
| orange | 60 seconds, Rimmel | nach 15 Sekunden Anlösezeit, dann schnell und vollständig | vollständig | vollständig |
| dunkelrot | Jade-Maybelline | sofort löslich, ohne Rückstände entfembar | verschmieren | vollständig, aber mit verschmieren |
| dunkelrot | NIVEA | vollständig | verschmieren | vollständig |
| weiß | Luminelle, Yves Rocher | nach 10 Sekunden Anlösezeit, dann innerhalb 5 Sekunden schnell und vollständig | fast vollständig | vollständig |

### Beispiel 6: Schmutzentferner

Hintergrund: Schmutzentfernung von verschiedenen Untergründen ist nach wie vor ein nur teilweise gelöstes Problem. In vielen Fällen schaffen es handelsübliche Reinigungsmittel nicht, in mikrometergroße Poren einzudringen und Schmutzpartikel heraus zu lösen oder anderweitig stark haftenden Schmutz abzulösen. Nanostrukturierte Flüssigkeiten dagegen vermögen dies, aufgrund der geringen Grenzflächenspannung, der Eigenschaft des Kriechens und der Neigung Nano- und Mikropartikel zu inkorporieren.

Als Beispiel wurde ein Abluftkasten gewählt, der über einen Zeitraum von ca. 10 Jahren im Freien die Ausscheidungen von Birkenbäumen (Birkenharz) auf seiner weißen Lackschicht aufgenommen hatte. Handelsübliche Reinigungsmittel erbrachten keinen Reinigungserfolg. Beide Mittel wurden 2 Minuten einwirken gelassen und anschließend mit feuchten Schwämmen abgespült.

Fig. 3 a) vorher, b) nach der Reinigung.

Bilder linke Seite (1): handelsübliches Tensid-Reinigungsmittel erbringen keinen Reinigungseffekt.

Bilder rechte Seite (2): nach der Reinigung mit der nanostrukturierten Formulierung ist das Birkenharz ohne Rückstände abgelöst worden.

Zusammensetzung des Fluids zur Reinigung (lisoCLEAR 55 DM - die Formulierung wurde für die Fliesen-, Keramik-, Fassaden- und Lackreinigung entwickelt):

| | | |
|---|---|---|
| Wasserphase: | Wasser | 55,28% |
| Ölphase: | Orangenterpen | 11,35% |
| Tensid: | Natriumdodecylsulfat | 8,80% |
| | C9-C11 Alkoholethoxylat (4) | 8,82% |
| Cotensid: | | |
| NP-MCA: | Diacetonalkohol (DAA) | 3,47% |
| | Ethylacetoacetat | 12,28% |
| | | 100,00% |

Schmutzentfernung von feinporigen, keramischen oder mineralischen Untergründen, wie Feinsteinfliesen, Rauputz, Beton, etc. ist nach wie vor ein nur teilweise gelöstes Problem. In vielen Fällen schaffen es handelsübliche Reinigungsmittel nicht, in mikrometergroße Poren einzudringen und Schmutzpartikel heraus zu lösen oder anderweitig stark haftenden Schmutz abzulösen. Nanophasenfluide dagegen vermögen dies aufgrund der geringen Grenzflächenspannung, der Eigenschaft des Kriechens und der Neigung Nano- und Mikropartikel zu inkorporieren.

Als Beispiel wurden Keramikfliesen eines 100 Jahre alten Jugendstil-Hauses gewählt, die über Jahrzehnte Schmutz aufgenommen hatten. Dieser Schmutz konnte mit handelsüblichen Reinigungsmitteln in den empfohlenen Dosierungen bei einer regelmäßigen wöchentlichen Reinigung nicht entfernt werden.

Alle untersuchten Mittel wurden genau 120 Sekunden einwirken gelassen und anschließend mit einem feuchten Schwamm abgewaschen. Danach wurde noch zwei Mal ohne Druck mit Wasser nachgereinigt. Trotz annähernd gleicher Inhaltsstoffe ergibt sich eine unterschiedliche Wirkung, je nach dem ob es sich um eine Mikroemulsion, eine konventionelle Emulsion oder um ein Nanophasenfluid handelt.

Die Ergebnisse sind in Fig. 8 dargestellt, wobei
a) den Zustand vor der Reinigung zeigt mit deutlich erkennbarem Schmutz in den Rillen der Fliesen und
b) den Zustand der gleichen Fliese nach der Reinigung zeigt.

Dabei wurden folgende Zusammensetzungen der Fluide zur Fliesen-Reinigung verwendet:
Zusammensetzung 1. ist eine Mikroemulsion, Zusammensetzung 2. ein Emulsionssystem und Zusammensetzung 3. das Nanophasensystem lisoCLEAR 55 nPMA- (die Formulierung wurde für die Fliesen-, Keramik-, Fassaden- und Lackreinigung entwickelt).

Im folgenden werden die Bestandteile der verwendeten Zusammensetzungen angegeben.

### Zusammensetzung 1. Mikroemulsion als Vergleichssystem zur Fliesenreinigung

| | | |
|---|---|---|
| Wasserphase: | Wasser | 55,58% |
| Ölphase: | Orangenterpen | 27,25% |
| Tensid: | Natriumdodecylsulfat | 2,50% |
| | C9-C11 Alkoholethoxylat(4) | 14,67% |
| Cotensid: | -- | |
| NP-MCA: | -- | |
| | | 100,00% |

### Zusammensetzung 2. Emulsionssystem als Vergleichssystem zur Fliesenreinigung

| | | |
|---|---|---|
| Wasserphase: | Wasser | 55,52% |
| | 1-Methyl-2-pyrrolidon | 1,85% |
| Ölphase: | Orangenterpen | 2,96% |
| Tensid: | Natriumdodecylsulfat | 23,09% |
| | | |
| Cotensid: | C9-C11 Alkoholethoxylat (4) | 7,03% |
| NP-MCA: | Ethylacetoacetat | 9,55% |
| | | 100,00% |

### Zusammensetzung 3. Nanophasenfluid lisoCLEAR 55 nPMA

| | | |
|---|---|---|
| Wasserphase: | Wasser | 55,28% |
| Ölphase: | Orangenterpen | 11,35% |
| Tensid: | Natriumdodecylsulfat | 8,80% |
| | C9-C11 Alkoholethoxylat (4) | 8,82% |
| Cotensid: | -- | |
| NP-MCA: | Ammonium-n-propylmaleamid (= Maleinsäuremono-n-propylamid Ammoniumsalz) | 3,47% |
| | Ethylacetoacetat | 12,28% |
| | | 100,00% |

### Beispiel 8: Kosmetikentferner

Hintergrund: wasserfeste, Kuss- und Tränenechte Kosmetika sind immer häufiger auf dem Markt zu finden und die Qualität ist in letzter Zeit zudem immer besser geworden. Solche Kosmetik lässt sich auch nicht einfach mehr mit warmem Wasser entfernen.

Viele Abschminkmittel bestehen aber unvorteilhaft aus zwei Phasen, die kurz vorher geschüttelt werden müssen, um eine Emulsion zu bilden. Nach häufig weniger als ein, zwei Minuten trennen sich die Phasen wieder auf (die Ölphase rahmt auf).

Folgendes Nanophasenfluid besteht aus sehr sanften, hautfreundlichen Inhaltsstoffen, die für sich einzeln wenn überhaupt eine nur schwache Reinigungswirkung besitzen. In Form eines nanostrukturierten Systems übertrifft die Wirkung allerdings die der handelsüblichen Produkte.

Fig. 4 links (1): a) vorher, b) nach der Reinigung mit Balea 2-Phasen Augen Make- up Entferner Waterproof.

Bild rechts (II): a) vorher, b) nach der Reinigung mit der nanostrukturierten Formulierung.

Die Zahlen ganz unten im Bild b) geben die Reinigungszyklen an, d.h. wie viel mal wurde mit einem Watte-Pad, der mit Reinigungsmittel getränkt worden war, mit leichtem Druck und nur in eine Richtung (hin zu den Fingern) über die Stelle gewischt.

### Zusammensetzung des Fluids zur Kosmetikentfernung ("Superformulierung"):

| | | |
|---|---|---|
| Wasserphase: | Wasser | 46,65% |
| | Natriumchlorid | 0,43% |
| | Glycerin | 0,47% |
| | Glycin | 0,35% |
| Ölphase: | Dicaprylylether | 17,76% |
| | Oleyloleat | 1,99% |
| Tensid: | Polyethylenglycol-7- Glycerylcocoat | 11,08% |
| | Polyoxyethylen(4)-sorbitanmonostearat | 7,82% |
| Cotensid: | - | 0,00% |
| NP-MCA: | Ethylacetoacetat | 12,25% |
| | Nicotinsäureamid | 0,31% |
| | Ascorbinsäure | 0,39% |
| | N-Acetylglycin | 0,50% |
| | | 100,00% |

### Beispiel 9: Haarentfärbung

Ein mit schwarzer Haarfarbe eingefärbter Haarbüschel wurden über Nacht (16,5 Stunden) in Fluid 42 aufbewahrt. Die entfärbten Haare (a) wurden mit dem unbehandelten Haarbüschel (b) verglichen: Ergebnisse sind in Fig. 5 dargestellt. Es zeigt sich, dass sich eine Entfärbung der behandelten Haare eingestellt hat.

### Zusammensetzung des Fluids V42:

| | | |
|---|---|---|
| Wasserphase: | Wasser | 28,48% |
| | Ethanol | 6,15% |
| Ölphase: | Orangenterpen | 20,49% |
| Tensid: | Cocoamidopropylbetain (Tego Betain CK D) | 6,05% |
| | C9-C11 Alkoholethoxylat (4) (Berol 260) | 15,37% |
| Cotensid: | 1-Hexanol | 2,15% |
| NP-MCA: | Ethylacetoacetat | 21,31 % |
| | | 100,00% |

### Beispiel 10: Nachweis der fluiden Nanophasen

Experimente zur Streuung eines Laserstrahls zum Nachweis der Nanostrukturierung in Nanophasensystemen

Ergebnisse sind in Fig. 6 dargestellt:
a) Ethylacetoacetat: grüner Laserstrahl nicht in der Flüssigkeit sichtbar, d.h. keine Streuung und daher keine Nanostrukturierung.
b) Aceton: grüner Laserstrahl nicht in der Flüssigkeit sichtbar, d.h. keine Streuung und daher keine Nanostrukturierung.
c) graffitiCRACK: grüner Laserstrahl ist sichtbar durch Streuung, d.h. die Flüssigkeit ist nanostrukturiert. Ein roter Laserstrahl wird im Übrigen kaum gestreut, da hier die Wellenlänge des roten Lichtes für eine Wechselwirkung zu groß ist.
d) lisoeLEAR: grüner Laserstrahl ist sichtbar durch Streuung, d.h. die Flüssigkeit ist nanostrukturiert.
e) Nagellackentferner (Rezept V113): grüner Laserstrahl ist sichtbar durch Streuung, d.h. die Flüssigkeit ist nanostrukturiert.

### Rezeptur zu c): Nanophasenfluid NP 2 (graffitiCRACK liquid):

| | | |
|---|---|---|
| Wasserphase: | Wasser | 21,04% |
| Ölphase: | Orangenterpen | 10,63% |
| | Benzylacetat | 10,52% |
| Tensid: | Natriumdodecylsulfat (SDS) | 13,18% |
| | C9-C11 Alkoholethoxylat (4) | 2,13% |
| Cotensid: | n-Hexanol | 10,52% |
| NP-MCA: | Triethylphosphat | 5,15% |
| | Ethylacetoacetat | 18,38% |
| | n-Butylacetat | 8,45% |
| | | 100,00% |

### Rezeptur zu d): IisoCLEAR 55 DAA (Hochleistungsreiniger für Fliesen, Fassaden, Steine, etc.

| | | |
|---|---|---|
| Wasserphase: | Wasser | 55,28% |
| | | |
| Ölphase: | Orangenterpen | 11,35% |
| | | |
| Tensid: | Natriumdodecylsulfat | 8,80% |
| | C9-C11 Alkoholethoxylat (4) | 8,82% |
| Cotensid: | | |
| NP-MCA: | Diacetonalkohol | 3,47% |
| | Ethylacetoacetat | 12,28% |
| | | |
| | | 100,00% |

### Rezeptur zu e): Nagellackentferner V113g

| | | |
|---|---|---|
| Wasserphase: | Wasser | 41,30% |
| Ölphase: | Dicaprylylether | 3,45% |
| | Ethylcinnamat | 0,42% |
| Tensid: | Natriumdodecylsulfat (SDS) | 4,20% |
| | | |
| | C13 Alkoholethoxylat (3) (Lutensol TO 3) | 9,81% |
| Cotensid: | 2-Phenylethanol | 3,46% |
| NP-MCA: | Diacetonalkohol | 11,74% |
| | Ethylacetoacetat | 25,62% |
| | | 100,00% |

### Beispiel 11: Vergleichsversuche Nanophasenfluide vs. Mikroemulsionen in einem Lackentfernungsexperiment

Für einen Vergleichstest im (Ab-)Löseverhalten von Lacken auf einem porösen Untergrund wurden 6 Flüssigkeiten getestet:
a) Ethylacetoacetat mit Wirkung als Lösungsmittel -nicht nanostrukturiert: Verschmieren der Lacke.
b) Lösungsmittel-Gemisch (V141) - nicht nanostrukturiert: Verschmieren der Lacke Glycerin: 8,34 %, Ethanol: 8,30 %, Diacetonalkohol: 35,02 %, Ethylacetoacetat: 21,00 %, n-Butylacetat: 6,64 %, Hexanol: 8,26 %, Benzylacetat: 8,30 %, Orangenterpen: 4,15 %.
c) Nanophasenfluid NP1 (V138d) - fast analog b) aber nanostrukturiert: kein Verschmieren, sondern Fragmentieren der Lacke
Wasserphase: Glycerin: 13,18 %, Ethanol: 13,76 %;
Ölphase: Dicaprylylether: 17,42 %;
Tensid: Natriumdodecylsulfat (SOS) 13,18 %, C9-C11 Alkoholethoxylat (4) 2,13 %;
Cotensid:
NP-MCA: Diacetonalkohol: 8,741 %, Acetylaceton: 8,71 %.

d) Nanophasenfluid NP 2 (graffitiCRACK liquid): Fragmentieren des Lacks, sehr gutes Ablösen des Lacks

| | | |
|---|---|---|
| Wasserphase: | Wasser: | 21,04 %; |
| Ölphase: | Orangenterpen: | 10,63 %, Benzylacetat: 10,52 %; |
| Tensid: | Natriumdodecylsulfat (SOS): 13,18 %, C9-C11 Alkoholethoxylat (4): 2,13 %; | |
| Cotensid: | | |
| NP-MCA: | n-Hexanol: 10,52 %; | |
| Triethylphosphat: | 5,15 %, Ethylacetoacetat: 18, 8 %, | |
| n-Butylacetat: | 8,45 %. | |

e) Nanophasenfluid NP 3 (V143)-fast analog f) aber Nanophasenfluid: stärkere Wirkung als f)

| | | |
|---|---|---|
| Wasserphase: | Wasser: | 27,11 %; |
| Ölphase: | Orangenterpen: | 24,46 %; |
| Tensid: | Natriumdodecylsulfat (SOS): | 8,92 %, C9-C11 Alkoholethoxylat (4): 21,77 %; |
| Cotensid: | | |
| NP-MCA: | Ethylacetoacetat: 1 | 7,75 %. |

f) Mikroemulsion ME 1 (V142): nanostrukturiert, aber als Mikroemulsion langsamer im Lackablösen

| | | |
|---|---|---|
| Wasserphase: | Wasser | 33,87 %; |
| Ölphase: | Orangenterpen | 34,21 %; |
| Tensid: | SOS | 11,34 %; |
| Cotensid: | Hexanol | 20,58 %) |

Die Flüssigkeiten wurden auf die poröse Rückseite einer Keramik-Platte aufgebracht, auf der Streifen folgender Lacke aufgebracht waren:
rot: Produkt Auto K (20330, VW/Audi, marsrot, L31B) gelb: Produkt Auto K (22218, FORD, Signalgelb, 77 KLP/97 grün: Produkt Auto K (21395, OPEL, mintgrün, 361)
silbern: Produkt Monex, Lack Spray (Rallye Felgensilber, 7093)
blau: Produkt Dupli Color, (sky blue, satin mat, DCP 5200 / RAL 5015)

Nach dem Aufbringen der Flüssigkeiten wurden sie 2 Minuten mit einem Pinsel einmassiert und anschließend mit fließendem Wasser abgewaschen.

Die Ergebnisse sind in Fig. 7 zusammengefasst. Die nicht nanostrukturierten Flüssigkeiten haben die Lacke zwar gelöst, aber auch deutlich verschmiert. Keines der nanostrukturierten Systemen zeigt dagegen ein Verschmieren.

Bei den nanostrukturierten Flüssigkeiten ist wiederum ein Unterschied zu erkennen, ob es sich um eine Mikroemulsion handelt oder um ein phasenaufgeweitetes System (Nanophasenfluid). Die zeitlich geringste Ablösefähigkeit zeigte die Mikroemulsion. Die deutlichste Wirkung hatte graffitiCRACK.

### Beispiel 12: weitere ausgewählte Beispielformulierungen für erfindungsgemäße Reinigungsmittel

### Formulierung 1:

| | Anteil [%] |
|---|---|
| Wasser | 19,80 |
| Triethylphosphat | 4,85 |
| Ethylacetoacetat | 17,30 |
| n-Butylacetat | 7,95 |
| | |
| 1-Hexanol | 9,90 |
| Benzylacetat | 9,90 |
| Orangenterpen | 10,00 |
| Natriumdodecylsulfat | 12,40 |
| C9-C11 | |
| Alkoholethoxylat (4) | 2,00 |
| Aerosil | 5,90 |

### Formulierung 2:

| | Anteil [%] |
|---|---|
| Wasser | 44,00 |
| N-Methyl - 2 - pyrrolidon | 4,50 |
| Ethylacetoacetat | 16,00 |
| Orangenterpen | 15,00 |
| Natriumdodecylsulfat | 11,50 |
| C9-C11 | |
| Alkoholethoxylat (4) | 9,00 |

### Formulierung 3:

| | Anteil [%] |
|---|---|
| Wasser | 55,28 |
| N-Methyl - 2 - pyrrolidon | 3,47 |
| Ethylacetoacetat | 12,28 |
| Orangenterpen | 11,35 |
| Natriumdodecylsulfat | 8,80 |
| C9-C11 | |
| Alkoholethoxylat (4) | 8,82 |

### Formulierung 4:

| | Anteil [%] |
|---|---|
| Wasser | 28,48 |
| Ethanol | 6,15 |
| Ethylacetoacetat | 21,31 |
| Orangenterpen | 20,49 |
| Hexanol | 2,15 |
| Tego Betain CK D | 6,05 |
| C9-C11 | |
| Alkoholethoxylat (4) | 15,37 |

### Formulierung 5:

| | Anteil [%] |
|---|---|
| Wasser | 41,32 |
| Diacetonalkohol | 11,73 |
| | |
| Ethylacetoacetat | 25,62 |
| Dicaprylylether | 3,45 |
| 2-Phenylethanol | 3,45 |
| Ethylcinnamat | 0,42 |
| C13 Alkoholethoxylat (3) | 9,8 |
| Natriumdodecylsulfat | 4,2 |

### Formulierung 6:

| | Anteil [%] |
|---|---|
| Wasser | 40 |
| Ethylacetoacetat | 4 |
| Orangenterpen | 36 |
| PEG 7 Glycoyl Cocoate | 16 |
| C9-C11 | |
| Alkoholethoxylat (4) | 4 |

### Formulierung 7:

| | Anteil [%] |
|---|---|
| Wasser | 38,8 |
| Diacetonalkohol | 2,40 |
| Orangenterpen | 38,8 |
| PEG 7 Glycoyl Cocoate | 16 |
| C9-C11 | |
| Alkoholethoxylat (4) | 4 |

### Formulierung 8:

| | Anteil [%] |
|---|---|
| Wasser | 40 |
| Acetylaceton | 4 |
| Orangenterpen | 36 |
| PEG 7 Glycoyl Cocoate | 16 |
| C9-C11 | |
| Alkoholethoxylat (4) | 4 |

### Formulierung 9:

| | Anteil [%] |
|---|---|
| Wasser | 61,36 |
| NaCl | 0,18 |
| EAA | 13,19 |
| Orangenterpen | 13,19 |
| C13 Alkoholethoxylat (8) | 4,59 |
| C13 Alkoholethoxylat (5) | 3,93 |
| Polyoxyethylen(20)-sorbitanmonostearat | 1,63 |
| | |
| Natrium-Dioctylsulfosuccinat | 1,93 |

### Formulierung 10:

| | Anteil [%] |
|---|---|
| Wasser | 6,43 |
| Ethylacetoacetat | 13,88 |
| Orangenterpen | 57,84 |
| Triethanolamin | 3,43 |
| Ölsäure | 18,42 |

### Formulierung 11:

| Anteil [%] | |
|---|---|
| Wasser | 46,65 |
| NaCl | 0,43 |
| Glycerin | 0,47 |
| Glycin | 0,35 |
| Ethylacetoacetat | 12,25 |
| Nicotinsäureamid | 0,31 |
| Ascorbinsäure | 0,39 |
| Acetylglycin | 0,50 |
| Dicaprylylether | 17,76 |
| Oleyloleat | 1,99 |
| PEG 7 Glycoyl Cocoate | 11,08 |
| Polyoxyethylen(4)-sorbitanmonostearat | 7,82 |
| | 100,00 |

## Patentansprüche

1. Reinigungsmittel, umfassend ein nicht koaleszierendes, thermodynamisch stabiles Nanophasensystem umfassend die Bestandteile:
a) 1-90 Gew.-% einer wasserunlöslichen Substanz (Öl) mit einer Löslichkeit in Wasser von weniger als 4g pro Liter;
b) 1 bis 80 Gew.-% einer amphiphilen Substanz, NP-MCA, die keine Tensidstruktur aufweist, alleine nicht strukturbildend ist, deren Löslichkeit in Wasser bzw. Öl zwischen 4g und 1000g pro Liter beträgt und die sich nicht bevorzugt an der Öl-Wasser-Grenzfläche anreichert, mit der Maßgabe, dass NP-MCA nicht ausgewählt ist aus 2-Ethyl-1,3-Hexandiol, 2-Methyl-2,4-Pentandiol, 2-(n-Butyl)-2-Ethyl-1,3-Propandiol und/oder aus 1,2-Diolen;
c) 2 bis 45 Gew.-% eines anionischen, kationischen, amphoteren und /oder nichtionischen Tensids und eines Cotensids mit hydrophil-lipophilen Molekülanteilen und ggf. Hilfsstoffe bis maximal 10 Gew.-%,
d) 1-90 Gew.-% Wasser und/oder ein wasserlösliches Lösungsmittel mit Hydroxyfunktionalität,
wobei sich die Prozentangaben auf das Gesamtgewicht des Reinigungsmittels beziehen,
**dadurch gekennzeichnet, dass** das NP-MCA bei einer Zugabe zu einem Öl-Wasser-Tensid-System enthaltend die Bestandteile a), c) und d) von 4 Gew.-% bezogen auf das Gesamtgewicht des Systems, dazu führt, dass sich in einem Phasendiagramm, welches den Verlauf der einphasigen und zweiphasigen und lamellaren Existenzbeeiche (lamellare Phase Lα-) des Systems in Abhängigkeit von der Tensidkonzentration und der Temperatur darsstellt (Fish-Diagramm oder "whale-Diagramm") die Fläche des im Phasendiagramm enthaltenen Dreiecks, das bestimmt ist durch die drei Eckpunkte:
i) den X-Punkt,
ii) den oberen Kreuzungspunkt des Grenzbereichs des einphasigen zum zweiphasigen Bereichs mit der parallel zur Temperaturordinate angelegten Tangente an das beginnende Lα-Gebiet und
iii) den unteren Kreuzungspunkt des Grenzbereichs des einphasigen zum zweiphasigen Bereichs mit der parallel zur Temperaturordinate angelegten Tangente an das beginnende Lα-Gebiet,
um mindestens 5% vergrößert; und
dass das NP-MCA ausgewählt ist aus:
a) Acetoacetaten der Formel II
C(R₃)₃-CO-CH₂-CO-O-R₄ [Formel II]
wobei
R₃ jeweils unabhängig voneinander Wasserstoff oder ein C₁ bis C₂ Alkyl ist und
R₄ ein verzweigtes oder unverzweigtes C₁ bis C₄ Alkyl ist;
oder
Acetoacetaten der Formel III
CH₃-CO-CH₂-CO-O-R₅ [Formel III]
wobei
R₅ ein C₁ bis C₄ Alkyl ist;
oder ausgewählt ist aus Ethylacetoacetat, iso-Propylacetoacetat, Methylacetoacetat, n-Butylacetoacetat, n-Propylacetoacetat oder tert-Butylacetoacetat.
b) Dionen der Formel IV
CH₃-(CH₂)ₚ-CO-(CH₂)_{q}-CO-(CH₂)ᵣ-CH₃ [Formel IV]
wobei
p, q, r unabhängig voneinander 0, 1 oder 2 sein können, mit der Maßgabe, dass, wenn die Summe aus p, q und r = 2 ist, die Verbindung gemäß Formel IV auch zyklisch sein kann (Cyclohexandion);
oder ausgewählt ist aus 2,3-Butandion (Diacetyl), 2,4-Pentandion (Acetylaceton), 3,4-Hexandion, 2,5-Hexandion, 2,3-Pentandion, 2,3-Hexandion, 1,4-Cyclohexandion oder 1,3-Cyclohexandion.
c) Estern der Formel V
R₆-CO-O-R₇ [Formel V]
wobei
R₆ eine Ringbindung zu R₇, CH₃ oder COCH₃ ist und
R₇ (CH₂)₂-O- Ringbindung zu R₆, oder (CH₂)₂-O-(CH₂)₃-CH₃ ist;
oder ausgewählt ist aus (2-Butoxyethyl)-acetat, Ethylencarbonat, Ethylpyruvat (2-Oxopropionsäureethylester),
d) Malein- bzw. Fumarsäureamiden der Formel VI
R₈-HN-CO-C=C-CO-O-R₉ [Formel VI]
wobei
R₈ Wasserstoff, ein verzweigtes oder unverzweigtes C₁ - C₄ Alkyl, oder ein verzweigtes oder unverzweigtes, lineares oder zyklisches C₁ - C₆ Alkyl ist, wobei das C₁ - C₆ Alkyl substituiert ist mit einer oder mehreren Gruppen ausgewählt aus OH, NH₂, COOH, CO, SO₃H, OP(OH)₂, und R₉ Wasserstoff oder ein verzweigtes oder unverzweigtes C1 - C4 Alkyl ist;
oder ausgewählt ist aus folgenden Maleinsäureamiden und deren Methyl-, Ethyl-, Propyl- und Butylester: N-Methylmaleamid; N-Ethylmaleamid; N-(n-Propyl)-maleamid; N-(i-Propyl)-maleamid; N-(n-Butyl)-maleamid; N(i-Butylmaleamid); N-(tert.-Butylmaleamid), sowie der entsprechenden Fumarsäureamide und deren Methyl-, Ethyl-, Propyl- und Butylester;
e) Diacetanalkohol (2-Methyl-2-pentanol-4-on).

2. Reinigungsmittel nach einem der vorhergehenden Ansprüche, **dadurch gekennzeichnet, dass** NP-MCA ausgewählt ist aus Acetoacetaten der Formel III
CH₃-CO-CH₂-CO-O-R₅ [Formel III]
wobei
R₅ ein C₁ bis C₄ Alkyl ist.

3. Reinigungsmittel nach einem der vorhergehenden Ansprüche, **dadurch gekennzeichnet, dass** das NP-MCA Ethylacetoacetat ist.

4. Reinigungsmittel nach einem der vorhergehenden Ansprüche, **dadurch gekennzeichnet, dass** die wasserunlösliche Substanz eine Wasserlöslichkeit von < 2 g pro Liter aufweist und die Substanz aus der Gruppe umfassend Alkane, Cykloalkane, Aromaten, langkettige Alkansäureester, Ester von Di- oder Tricarbonsäuren, Terpene, oder deren Mischungen ausgewählt ist.

5. Verfahren zur Entfernung unerwünschter Farben und Lacke von Oberflächen, **dadurch gekennzeichnet, dass** ein Reinigungsmittel nach einem der Ansprüche 1 bis 4 auf die unerwünschte Farbe oder den Lack aufgebracht wird, einwirkt und anschließend die Farbe oder der Lack mit Wasser entfernt wird, wobei die Einwirkzeit beim Graffitientferner von etwa 10 Sekunden bis etwa 30 Minuten beträgt, beim Abbeizer von etwa 20 Minuten bis etwa 3 Stunden und beim Nagellackentfernen von etwa 3 bis etwa 30 Sekunden.

6. Verwendung des Reinigungsmittels nach den Ansprüchen 1 bis 4 als Graffitientferner, Abbeizer, Nagellackentferner, Schmutzentferner, Fliesenreiniger, Kosmetikentferner oder Haarentfärber.

## Claims

1. A cleaning composition comprising a non-coalescing, thermodynamically stable nanophase system comprising the ingredients:
a) 1-90% by weight of a water-insoluble substance (oil) having a solubility in water of less than 4g per liter;
b) 1 to 80 wt. % by weight of an amphiphilic substance, NP-MCA, which has no surfactant structure, is not structure-forming on its own, whose solubility in water or oil is between 4g and 1000g per liter and which does not preferentially accumulate at the oil-water interface, with the proviso that NP-MCA is not selected from 2-ethyl-1,3-hexanediol, 2-methyl-2,4-pentanediol, 2-(n-butyl)-2-ethyl-1,3-propanediol and/or from 1,2-diols;
c) 2 to 45% by weight of an anionic, cationic, amphoteric and/or non-ionic surfactant and a cosurfactant with hydrophilic-lipophilic molecular components and, if appropriate, auxiliaries up to a maximum of 10% by weight,
d) 1-90% by weight of water and/or a water-soluble solvent with hydroxy functionality, the percentages being based on the total weight of the cleaning agent,
**characterized in that** the NP-MCA, when added to an oil-water-surfactant system containing the components a), c) and d) of 4 wt. % based on the total weight of the system, results in the area of the triangle contained in the phase diagram, which is determined by the three corner points, changing in a phase diagram which shows the course of the single-phase and two-phase and lamellar existence ranges (lamellar phase Lα-) of the system as a function of the surfactant concentration and the temperature (fish diagram or "whale diagram"):
i) the X point,
ii) the upper crossing point of the boundary region of the single-phase to the two-phase region with the tangent to the starting Lα region parallel to the temperature coordinate and
iii) the lower crossing point of the boundary region of the single-phase to the two-phase region with the tangent to the starting Lα region parallel to the temperature coordinate, is increased by at least 5%; and that the NP-MCA is selected from:
(a) acetoacetates of formula II
C(R3)3-CO-CH2-CO-O-R4 [formula II]
wherein
R3 is independently hydrogen or a C1 to
C2 alkyl and
R4 is a branched or unbranched C1 to C4 alkyl;
or
an acetoacetate of formula III
CH3-CO-CH2-CO-O-R5 [formula III]
wherein
R5 is a C1 to C4 alkyl;
or is selected from ethyl acetoacetate, iso-propyl acetoacetate, methyl acetoacetate, n-butyl acetoacetate, n-propyl acetoacetate or tert-butyl acetoacetate.
(b) Diones of formula IV
CH3-(CH2)p-CO-(CH2)q-CO-(CH2)r-CH3 [formula IV]
where
p, q, r may independently of one another be 0, 1 or 2, with the proviso that if the sum of p, q and r = 2, the compound according to formula IV may also be cyclic (cyclohexanedione);
or is selected from 2,3-butanedione (diacetyl), 2,4-pentanedione (acetylacetone), 3,4-hexanedione, 2,5-hexanedione, 2,3-pentanedione, 2,3-hexanedione, 1,4-cyclohexanedione or 1,3-cyclohexanedione.
(c) Esters of the formula V
R6-CO-O-R7 [formula V]
wherein
R6 is a ring bond to R7, CH3 or COCH3 and
R7 is (CH2)2-O- ring bond to R6, or (CH2)2-O-(CH2)3-CH3;
or is selected from (2-butoxyethyl) acetate, ethylene carbonate, ethyl pyruvate (2-oxopropionic acid ethyl ester),
(d) maleic or fumaric acid amides of formula VI
R8-HN-CO-C=C-CO-O-R9 [formula VI]
wherein
R8 is hydrogen, a branched or unbranched C1 - C4 alkyl, or a branched or unbranched, linear or cyclic C1 - C6 alkyl, wherein the C1 - C6 alkyl is substituted with one or more groups selected from OH, NH2, COOH, CO, SO3H, OP(OH)2, and R9 is hydrogen or a branched or unbranched C1 - C4 alkyl;
or is selected from the following maleic acid amides and their methyl, ethyl, propyl and butyl esters: N-methylmaleamide; N-ethylmaleamide; N-(n-propyl)-maleamide; N-(i-propyl)-maleamide; N-(n-butyl)-maleamide; N(i-butylmaleamide); N-(tert. butylmaleamide), as well as the corresponding fumaric acid amides and their methyl, ethyl, propyl and butyl esters;
(e) diaceton alcohol (2-methyl-2-pentanol-4-one).

2. Cleaning agent according to one of the preceding claims, **characterized in that** NP-MCA is selected from acetoacetates of the formula III
CH3-CO-CH2-CO-O-R5 [formula III]
wherein
R5 is a C1 to C4 alkyl.

3. Cleaning agent according to one of the preceding claims, **characterized in that** the NP-MCA is ethyl acetoacetate.

4. Cleaning agent according to one of the preceding claims, **characterized in that** the water-insoluble substance has a water solubility of < 2 g per liter and the substance is selected from the group comprising alkanes, cycloalkanes, aromatics, long-chain alkanoic acid esters, esters of di- or tricarboxylic acids, terpenes, or mixtures thereof.

5. Process for removing undesirable paints and lacquers from surfaces, **characterized in that** a cleaning agent according to one of claims 1 to 4 is applied to the undesirable paint or lacquer, is allowed to act and then the paint or lacquer is removed with water, wherein the exposure time for the graffiti remover is from about 10 seconds to about 30 minutes, for the paint stripper from about 20 minutes to about 3 hours and for the nail polish remover from about 3 to about 30 seconds.

6. Use of the cleaning agent according to claims 1 to 4 as a graffiti remover, paint stripper, nail polish remover, dirt remover, tile cleaner, cosmetic remover or hair dye remover.

## Revendications

1. Détergent comprenant un système nanophasique non coalescent, thermodynamiquement stable, comprenant les ingrédients:
a) 1-90% en poids d'une substance insoluble dans l'eau (huile) ayant une solubilité dans l'eau inférieure à 4g par litre;
b) 1 à 80 % en poids de % d'une substance amphiphile, NP-MCA, qui ne présente pas de structure tensioactive, n'est pas structurante à elle seule, dont la solubilité dans l'eau ou l'huile est comprise entre 4g et 1000g par litre et qui ne s'accumule pas préférentiellement à l'interface huile-eau, à condition que NP-MCA ne soit pas choisi parmi le 2-éthyl-1,3-hexanediol, le 2-méthyl-2,4-pentanediol, le 2-(n-butyl)-2-éthyl-1,3-propanediol et/ou les 1,2-diols;
c) 2 à 45 % en poids d'un agent tensioactif anionique, cationique, amphotère et/ou non ionique et d'un co-tensioactif avec des parts de molécules hydrophiles-lipophiles et éventuellement des adjuvants jusqu'à 10 % en poids au maximum,
d) 1-90 % en poids d'eau et/ou d'un solvant hydrosoluble à fonctionnalité hydroxyle, les pourcentages se rapportant au poids total du produit de nettoyage,
**caractérisé en ce que** le NP-MCA est utilisé lors d'une addition à un système tensioactif huile-eau contenant les composants a), c) et d) de 4 % en poids. % par rapport au poids total du système, la surface du triangle contenu dans le diagramme de phases, qui est déterminé par les trois points d'angle, s'agrandit dans un diagramme de phases qui représente l'évolution des rayons d'existence à une phase et à deux phases et lamellaires (phase lamellaire La-) du système en fonction de la concentration en agent tensioactif et de la température (diagramme de poisson ou « diagramme de baleine ») :
i) le point X,
ii) le point d'intersection supérieur de la zone limite de la zone monophasée à la zone diphasée avec la tangente à la zone La naissante, parallèle à l'ordonnée à la température, et
iii) le point d'intersection inférieur de la limite entre la zone monophasée et la zone biphasée avec la tangente à la zone La de départ, parallèle à la coordonnée de température, est augmentée d'au moins 5% ; et **en ce que** le NP-MCA est choisi parmi :
(a) des acétoacétates de formule Il
C(R3)3-CO-CH2-CO-O-R4 [formule II].
où
chaque R3 est indépendamment un hydrogène ou un alkyle en C1 à
C2 alkyle et
R4 est un alkyle en C1 à C4 ramifié ou non ramifié;
ou
des acétoacétates de formule III
CH3-CO-CH2-CO-O-R5 [formule III].
où
R5 est un alkyle en C1 à C4;
ou est choisi parmi l'acétoacétate d'éthyle, l'acétoacétate d'isopropyle, l'acétoacétate de méthyle, l'acétoacétate de n-butyle, l'acétoacétate de n-propyle ou l'acétoacétate de tert-butyle.
(b) les diones de formule IV
CH3-(CH2)p-CO-(CH2)q-CO-(CH2)r-CH3 [formule IV].
où
p, q, r peuvent être indépendamment les uns des autres 0, 1 ou 2, à condition que, lorsque la somme de p, q et r = 2, le composé selon la formule IV puisse également être cyclique (cyclohexanedione);
ou est choisi parmi la 2,3-butanedione (diacétyle), la 2,4-pentanedione (acétylacétone), la 3,4-hexanedione, la 2,5-hexanedione, la 2,3-pentanedione, la 2,3-hexanedione, la 1,4-cyclohexanedione ou la 1,3-cyclohexanedione.
(c) les esters de formule V
R6-CO-O-R7 [formule V].
dans laquelle
R6 est une liaison cyclique à R7, CH3 ou COCH3 et
R7 est une liaison cyclique (CH2)2-O- avec R6, ou (CH2)2-O-(CH2)3-CH3 ;
ou est choisi parmi l'acétate de (2-butoxyéthyle), le carbonate d'éthylène, le pyruvate d'éthyle (ester éthylique de l'acide 2-oxopropionique),
(d) les amides de l'acide maléique ou fumarique de formule VI
R8-HN-CO-C=C-CO-O-R9 [formule VI].
dans laquelle
R8 est un hydrogène, un alkyle en C1 à C4 ramifié ou non ramifié, ou un alkyle en C1 à C6 ramifié ou non ramifié, linéaire ou cyclique, l'alkyle en C1 à C6 étant substitué par un ou plusieurs groupes choisis parmi OH, NH2, COOH, CO, SO3H, OP(OH)2, et R9 est un hydrogène ou un alkyle en C1 à C4 ramifié ou non ramifié;
ou est choisi parmi les amides de l'acide maléique suivants et leurs esters méthylique, éthylique, propylique et butylique: N-méthylmaléamide N-éthylmaléamide ; N-(n-propyl)-maléamide ; N-(i-propyl)-maléamide ; N-(n-butyl)-maléamide ; N(i-butylmaléamide) ; N-(tert. butylmaléamide), ainsi que les amides d'acide fumarique correspondants et leurs esters méthylique, éthylique, propylique et butylique;
(e) l'alcool diacétique (2-méthyl-2-pentanol-4-one).

2. Détergent selon l'une des revendications précédentes, **caractérisé en ce que** le NP-MCA est choisi parmi les acétoacétates de formule III
CH3-CO-CH2-CO-O-R5 [formule III].
où
R5 est un alkyle en C1 à C4.

3. Détergent selon l'une des revendications précédentes, **caractérisé en ce que** le NP-MCA est l'acétoacétate d'éthyle.

4. Détergent selon l'une des revendications précédentes, **caractérisé en ce que** la substance insoluble dans l'eau présente une solubilité dans l'eau < 2 g par litre et la substance est choisie dans le groupe comprenant les alcanes, les cycloalcanes, les aromatiques, les esters d'acides alcanoïques à longue chaîne, les esters d'acides di- ou tricarboxyliques, les terpènes, ou leurs mélanges.

5. Procédé pour éliminer des peintures et des vernis indésirables de surfaces, **caractérisé en ce qu'**un agent de nettoyage selon l'une des revendications 1 à 4 est appliqué sur la peinture ou le vernis indésirable, agit et ensuite la peinture ou le vernis est éliminé avec de l'eau, le temps d'action étant d'environ 10 secondes à environ 30 minutes pour le décapant de graffiti, d'environ 20 minutes à environ 3 heures pour le décapant de vernis à ongles et d'environ 3 à environ 30 secondes pour le décapant de vernis à ongles.

6. Utilisation du produit de nettoyage selon les revendications 1 à 4 en tant que décapant pour graffiti, décapant pour vernis à ongles, décapant pour salissures, nettoyant pour carrelage, décapant pour cosmétiques ou décolorant pour cheveux.
